# EUROPEAN PATENT SPECIFICATION

(11) **EP 4 100 010 B1**
(45) Date of publication and mention of the grant of the patent: **07.01.2026**
(21) Application number: 21750608.8
(22) Date of filing: 08.02.2021
(51) Int. Cl.: A61K 31/445, A61K 31/45, A61K 31/452, C07D 211/94, A61P 25/36, A61P 11/00, A61P 9/02, A61K 31/40, A61K 31/4468, A61K 31/4535

(54) **NITROXIDE COMPOUNDS FOR USE IN THE TREATMENT OF OPIOID-INDUCED RESPIRATORY DEPRESSION (OIRD)**
NITROXIDVERBINDUNGEN ZUR VERWENDUNG BEI DER BEHANDLUNG VON OPIOIDINDUZIERTER ATEMDEPRESSION (OIRD)
COMPOSÉS NITROXYDIQUES À UTILISER DANS LE TRAITEMENT DE LA DÉPRESSION RESPIRATOIRE INDUITE PAR LES OPIOÏDES (OIRD)

(30) Priority: 07.02.2020 US 202062971675 P
(43) Date of publication of application: 14.12.2022
(73) Proprietor: Case Western Reserve University, Cleveland, OH 44106 (US)
(72) Inventor: LEWIS, Stephen J., Cleveland, Ohio 44106 (US); SECKLER, James M., Cleveland, Ohio 44106 (US)
(74) Representative: Maiwald GmbH
(86) International application number: PCT/US2021/017088
(87) International publication number: WO 2021/159077

(56) References cited:
- WO-A1-2018/093666
- US-A1- 2009 062 338
- US-A1- 2017 151 230
- US-A1- 2019 358 189
- ALGERA MARIJKE HYKE ET AL: "Opioid-induced respiratory depression in humans: a review of pharmacokinetic-pharmacodynamic modelling of reversal", BRITISH JOURNAL OF ANAESTHESIA, vol. 122, no. 6, 1 June 2019 (2019-06-01), pages e168 - e179, XP093112583, ISSN: 0007-0912, DOI: 10.1016/j.bja.2018.12.023

## Description

### TECHNICAL FIELD

Embodiments described herein relate to compositions for attenuating and/or inhibiting opioid induced respiratory depression in a subject in need thereof, and particularly relates to compositions for treating respiratory depression associated with opioid use.

### BACKGROUND

Commonly used opioid medications suppress ventilatory drive. Specifically, they depress the slope of the relationship between PCO₂ and minute ventilation. This is a major issue in several important settings. In the operating room and post-anesthesia care setting, patients may have prolonged respiratory depression associated with pain control. This results in prolonged hospitalizations or early, risky discharge and death. In the chronic pain population - in the Veteran's Administration system, for example - death from nocturnal respiratory depression is at epidemic proportions among patients on chronic opiate therapy. Opiate addiction is also at epidemic levels, and hundreds of young people die annually without an effective emergency respiratory stimulant. On the battlefield, medics have to choose between excruciating pain and risk of death from respiratory depression. In the Intensive Care population, physicians often have to choose between the risk of being on the ventilator for one or more days and the risk of awaking a patient in pain and distress. This is a problem in patients with a baseline blunted CO₂ response, such as patients with severe chronic obstructive pulmonary disease (COPD), cystic fibrosis (CF) or other obstructive lung disease.

Fentanyl is high-potency opioid receptor (OR) agonist that is widely used to treat both acute and chronic pain. The misuse/abuse of fentanyl and analogues, such as sufentanil and carfentanil, leads to adverse consequences, including often lethal depression of ventilation. Fentanyl is thought of as a selective µ-OR agonist and has very high affinity for µ-ORs. However, fentanyl also activates δ- and κ-ORs with affinities and intrinsic activities of biological significance. For example, whereas fentanyl has low affinity for κ-ORs it has a remarkably high efficacy at these receptors. The mechanisms responsible for the ventilatory depressant and analgesic effects of fentanyl and analogues have been studied extensively. Pre-treatment of rats with naloxone methiodide, a peripherally-restricted µ-OR antagonist, attenuated fentanyl-induced analgesia, decreases in tidal volume (TV) and increases in Alveolar-arterial (A-a) gradient that were indicative of ventilation-perfusion mismatch or shunting in the lungs. Accordingly, it is likely that the pharmacological actions of fentanyl involve a mixture of effects in the periphery (e.g., vagal cardiopulmonary afferents, the chest-wall and carotid bodies), brain regions, such as the area postrema that are devoid of a blood-brain barrier, and also brain structures within the blood brain barrier, such as the nucleus tractus solitarius.

M. H. Algera et al. describe in "Opioid-induced respiratory depression in humans: a review of pharmacokinetic-pharmacodynamic modelling of reversal", British Journal of Anaesthesia, 2019, 122, 6, e168-e179, human studies on reversal of opioid-induced respiratory depression (OIRD) using models that describe and predict the time course of pharmacokinetics (PK) and pharmacodynamics (PD) of opioids and reversal agents and link PK to PD. Doing so, M. H. Algera et al. describe compounds for use in the treatment of OIRD such as naloxone, ketamine, and doxapram.

### SUMMARY

Embodiments described herein relate to a composition comprising a nitroxide compound for use in the treatment of opioid induced respiratory depression in a subject in need thereof, by administering to said subject a therapeutically effective amount of said composition comprising a nitroxide compound, wherein the nitroxide compound comprises at least one of 4-hydroxy-2,2,6,6-tetramethylpiperidine-1-oxyl (Tempol), 2,2,6,6-tetramethylpiperidine-1-oxyl (Tempo), 4-amino-2,2,6,6-tetramethyl-1-piperidinyloxy (Tempamine); 4-oxo-2,2,6,6-tetramethyl-1-piperidinyloxy (4-oxo-Tempo), 4-carboxy-2,2,6,6-tetramethyl-1-piperidinyloxy (4-carboxy-Tempo), 4-acetamido-2,2,6,6-tetramethyl-1-piperidinyloxy, 3-aminomethyl- 2,2,5,5-tetramethyl-1 -pyrrolidinyl-N-oxyl (3-aminomethyl-Proxyl), 3-cyano-2,2,5,5- tetramethyl-1-pyrrolidinyl-N-oxyl (3-cyano-Proxyl), 3-Carbamoyl-2,2,5,5-tetramethyl-1- pyrrolidinyl-N-oxyl (3-Carbamoyl-Proxyl), 3-Carboxy-2,2,5,5-tetramethyl-1-pyrrolidinyl-N- oxyl (3-Carboxy-Proxyl),or pharmaceutically acceptable salts, tautomers, or solvates thereof.. It was found that administration of such nitroxide compounds, such as Tempol, dose-dependently and markedly attenuated the respiratory depressant effects elicited by opioids, such as fentanyl, without affecting opioid-induced analgesia.

In some embodiments, the treatment of opioid induced respiratory depression in a subject in need thereof is attenuation of opioid induced respiratory depression in a subject in need thereof, and the therapeutically effective amount of said composition comprising a nitroxide compound is an amount effective to attenuate the opioid induced respiratory depression.

In some embodiments, the amount or therapeutically effective amount is an amount effective to increase tidal volume, increase respiratory frequency, increase minute ventilation, increase mean arterial blood pressure, increase diastolic blood pressure, and/or increase systolic blood pressure in the subject.

The composition can be administered to the subject systemically by, for example, topical *(e.g.,* inhalation), enteral *(e.g.,* oral), and/or parenteral *(e.g.,* intravenous injection) administration.

The nitroxide compound includes at least one of a pyrrolidine nitroxide, piperidine nitroxide, or oxazolidine nitroxide, namely, the nitroxide compound includes at least one of 4-hydroxy-2,2,6,6-tetramethylpiperidine-1-oxyl (Tempol), 2,2,6,6-tetramethylpiperidine-1-oxyl (Tempo), 4-amino-2,2,6,6-tetramethyl-1-piperidinyloxy (Tempamine); 4-oxo-2,2,6,6-tetramethyl-1-piperidinyloxy (4-oxo-Tempo), 4-carboxy-2,2,6,6-tetramethyl-1-piperidinyloxy (4-carboxy-Tempo), 4-acetamido-2,2,6,6-tetramethyl-1-piperidinyloxy, 2-ethyl-2,5,5-trimethyl-3-oxazolidine-1-oxyl (Oxano), 3-aminomethyl-2,2,5,5-tetramethyl-1 -pyrrolidinyl-N-oxyl (3-aminomethyl-Proxyl), 3-cyano-2,2,5,5-tetramethyl-1-pyrrolidinyl-N-oxyl (3-cyano-Proxyl), 3-Carbamoyl-2,2,5,5-tetramethyl-1-pyrrolidinyl-N-oxyl (3-Carbamoyl-Proxyl), 3-Carboxy-2,2,5,5-tetramethyl-1-pyrrolidinyl-N-oxyl (3-Carboxy-Proxyl),or pharmaceutically acceptable salts, tautomers, or solvates thereof.

In still other embodiments, the nitroxide compound can include 4-hydroxy-2,2,6,6-tetramethylpiperidine-1-oxyl (Tempol) or pharmaceutically acceptable salts, tautomers, or solvates thereof.

In some embodiments, the opioid can include at least one of alfentanil, buprenorphine, butorphanol, codeine, diamorphine, dextromoramide, dezocine, dihydrocodeine, carfentanil, fentanyl, hydrocodone, hydromorphone, levorphanol, meperidine, meptazinol, methadone, morphine, nalbuphine, nalorphine, opium, oxycodone, oxymorphone, pentazocine, propoxyphene, remifentanil, sufentanil, tapentadol, and tramadol, and pharmaceutically acceptable salts thereof. For example, the opioid can be carfentanil, fentanyl, remifentanil, or sufentanil.

In other embodiments, the composition can be administered to the subject at an amount effective to prevent the need for mechanical ventilation in subjects with acutely impaired ventilatory and/or respiratory drive because of an acute requirement for narcotic analgesia.

In still other embodiments, the composition can be administered to a subject in combination with at least one additional therapeutic agent that changes normal breathing in a subject. The additional agent can be selected from the group consisting of an opioid, doxapram and enantiomers thereof, acetazolamide, almitrine, theophylline, caffeine, methylprogesterone and related compounds, sedatives that decrease arousal threshold in sleep disordered breathing patients, sodium oxybate, benzodiazepine receptor agonists, orexin antagonists, tricyclic antidepressants, serotonergic modulators, adenosine and adenosine receptor and nucleoside transporter modulators, cannabinoids, orexins, melatonin agonists, ampakines, and combinations thereof.

In yet another embodiment, the composition and the agent are separately administered to the subject. In yet another embodiment, the compound and the agent are co-administered to the subject.

Still other embodiments described herein relate to a composition that includes an opioid capable of inducing respiratory depression in a subject and an amount of nitroxide compound effective to attenuate the opioid induced respiratory depression when the composition is administered to the subject, wherein the nitroxide compound comprises at least one of 4-hydroxy-2,2,6,6-tetramethylpiperidine-1-oxyl (Tempol), 2,2,6,6-tetramethylpiperidine-1-oxyl (Tempo), 4-amino-2,2,6,6-tetramethyl-1-piperidinyloxy (Tempamine); 4-oxo-2,2,6,6-tetramethyl-1-piperidinyloxy (4-oxo-Tempo), 4-carboxy-2,2,6,6-tetramethyl-1-piperidinyloxy (4-carboxy-Tempo), 4-acetamido-2,2,6,6-tetramethyl-1-piperidinyloxy, 3-aminomethyl- 2,2,5,5-tetramethyl-1 -pyrrolidinyl-N-oxyl (3-aminomethyl-Proxyl), 3-cyano-2,2,5,5- tetramethyl-1-pyrrolidinyl-N-oxyl (3-cyano-Proxyl), 3-Carbamoyl-2,2,5,5-tetramethyl-1- pyrrolidinyl-N-oxyl (3-Carbamoyl-Proxyl), 3-Carboxy-2,2,5,5-tetramethyl-1-pyrrolidinyl-N- oxyl (3-Carboxy-Proxyl),or pharmaceutically acceptable salts, tautomers, or solvates thereof.

In some embodiments, the opioid can include at least one of alfentanil, buprenorphine, butorphanol, carfentanil, codeine, diamorphine, dextromoramide, dezocine, dihydrocodeine, fentanyl, hydrocodone, hydromorphone, levorphanol, meperidine, meptazinol, methadone, morphine, nalbuphine, nalorphine, opium, oxycodone, oxymorphone, pentazocine, propoxyphene, remifentanil, sufentanil, tapentadol, and tramadol, and pharmaceutically acceptable salts thereof. For example, the opioid can be carfentanil, fentanyl, remifentanil, or sufentanil.

The nitroxide compound includes at least one of a pyrrolidine nitroxide, piperidine nitroxide, or oxazolidine nitroxide, namely, the nitroxide compound includes at least one of 4-hydroxy-2,2,6,6-tetramethylpiperidine-1-oxyl (Tempol), 2,2,6,6-tetramethylpiperidine-1-oxyl (Tempo), 4-amino-2,2,6,6-tetramethyl-1-piperidinyloxy (Tempamine); 4-oxo-2,2,6,6-tetramethyl-1-piperidinyloxy (4-oxo-Tempo), 4-carboxy-2,2,6,6-tetramethyl-1-piperidinyloxy (4-carboxy-Tempo), 4-acetamido-2,2,6,6-tetramethyl-1-piperidinyloxy, 2-ethyl-2,5,5-trimethyl-3-oxazolidine-1-oxyl (Oxano), 3-aminomethyl-2,2,5,5-tetramethyl-1 -pyrrolidinyl-N-oxyl (3-aminomethyl-Proxyl), 3-cyano-2,2,5,5-tetramethyl-1-pyrrolidinyl-N-oxyl (3-cyano-Proxyl), 3-Carbamoyl-2,2,5,5-tetramethyl-1-pyrrolidinyl-N-oxyl (3-Carbamoyl-Proxyl), 3-Carboxy-2,2,5,5-tetramethyl-1-pyrrolidinyl-N-oxyl (3-Carboxy-Proxyl),or pharmaceutically acceptable salts, tautomers, or solvates thereof.

In still other embodiments, the nitroxide compound can include 4-hydroxy-2,2,6,6-tetramethylpiperidine-1-oxyl (Tempol) or pharmaceutically acceptable salts, tautomers, or solvates thereof.

### BRIEF DESCRIPTION OF THE DRAWINGS

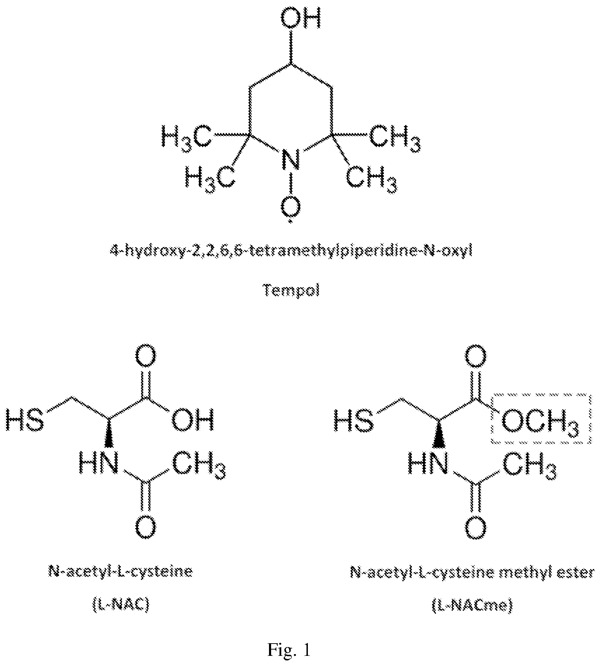

Fig. 1 illustrates structures of Tempol, N-acetyl-L-cysteine and N-acetyl-L-cysteine methyl ester.

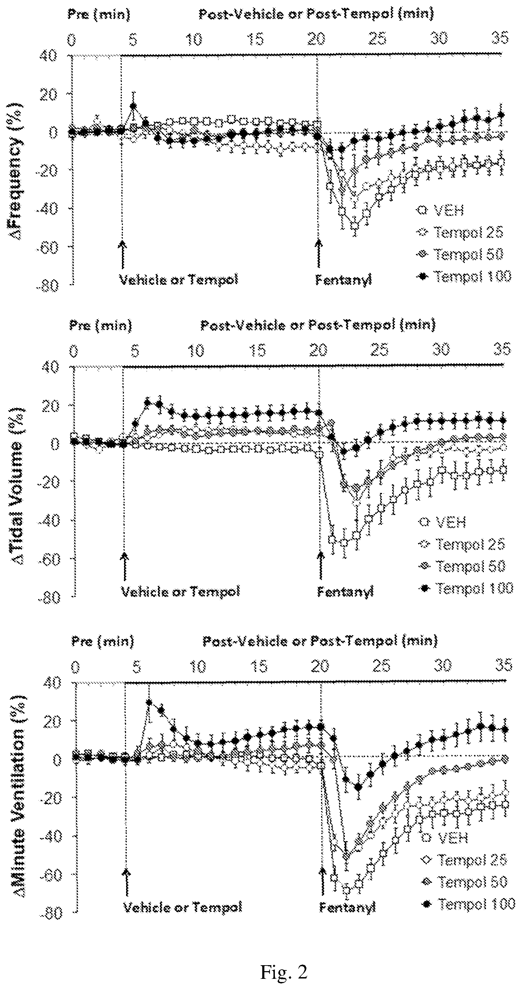

Fig. 2 illustrates a summary of the changes in ventilatory parameters elicited by bolus injections of vehicle or Tempol (25, 50 or 100 mg/kg, IV) and subsequent injections of fentanyl (5 µg/kg, IV) in isoflurane-anesthetized rats. There were 12 rats in the vehicle group, and 3, 5 and 12 rats in the 25, 50 and 100 mg/kg Tempol groups, respectively. The data are presented as mean ± SEM.

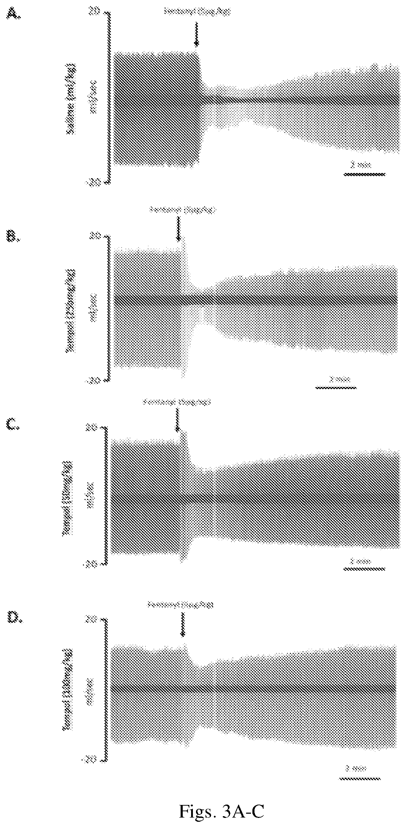

Fig. 3 illustrates typical examples of the effects of an injection of fentanyl (5 µg/kg, IV) on ventilatory waveforms recorded from 4 separate rats that had received an IV injection of vehicle (saline, Panel A) or tempol at 25 mg/kg (Panel B), 50 mg/kg (Panel C) or 100 mg/kg (Panel D).

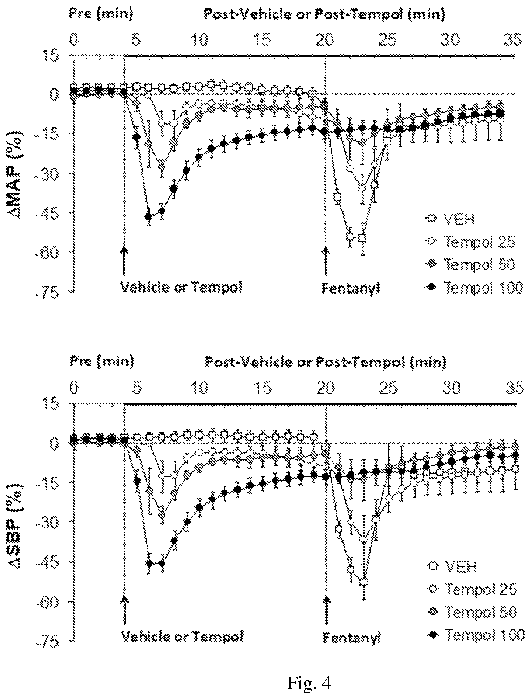

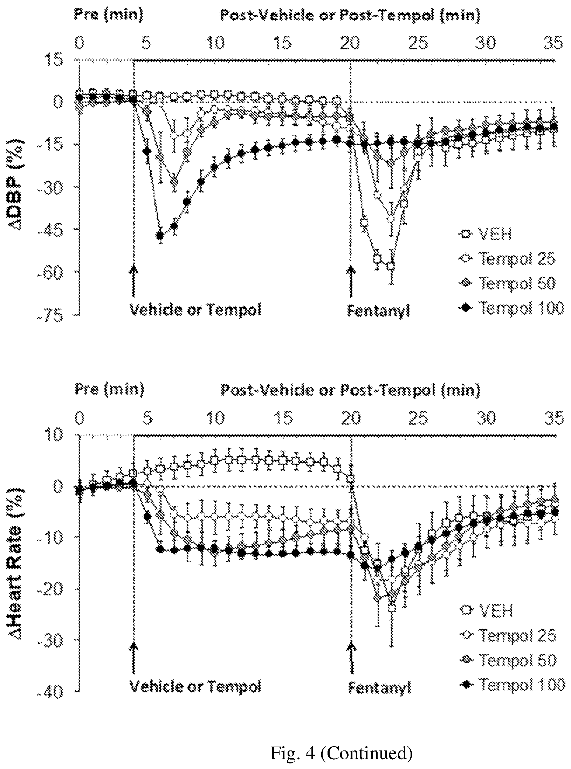

Fig. 4 illustrates a summary of the changes in mean (MAP), diastolic (DBP) and systolic (SBP) arterial blood pressures and heart rate elicited by bolus injections of vehicle or Tempol (25, 50 or 100 mg/kg, IV) and subsequent injections of fentanyl (5 µg/kg, IV) in isoflurane-anesthetized rats. There were 12 rats in the vehicle group, and 3, 5 and 12 rats in the 25, 50 and 100 mg/kg Tempol groups, respectively. The data are presented as mean ± SEM.

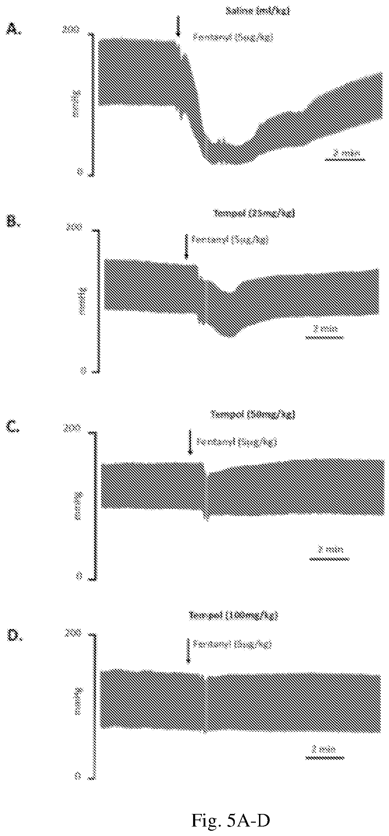

Fig. 5 illustrates typical examples of the effects of an injection of fentanyl (5 µg/kg, IV) on arterial blood pressure waveform recorded from 4 separate rats that had received an IV injection of vehicle (saline, Panel A) or tempol at 25 mg/kg (Panel B), 50 mg/kg (Panel C) or 100 mg/kg (Panel D).

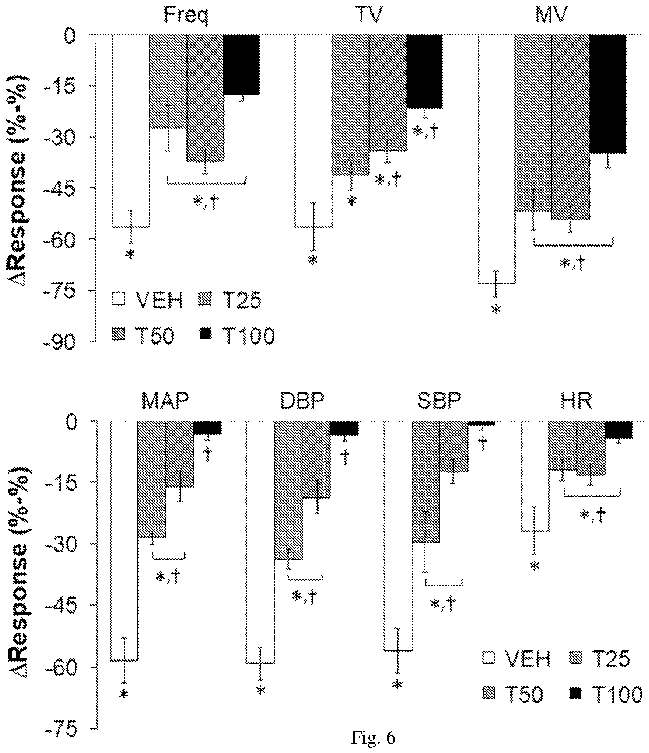

Fig. 6 illustrates a summary of the peak changes in ventilatory parameters (top panel) and cardiovascular parameters (bottom panel) elicited by bolus injections of vehicle (VEH) or Tempol (25, 50 or 100 mg/kg, IV, designated T25, T50 and T100) and subsequent injections of fentanyl (5 µg/kg, IV) in isoflurane-anesthetized rats. There were 12 rats in the vehicle group, and 3, 5 and 12 rats in the 25, 50 and 100 mg/kg Tempol-treated groups, respectively. Data are presented as mean ± SEM. *P < 0.05, significant change. ^{†}P < T25, T50 and/or T100 versus vehicle.

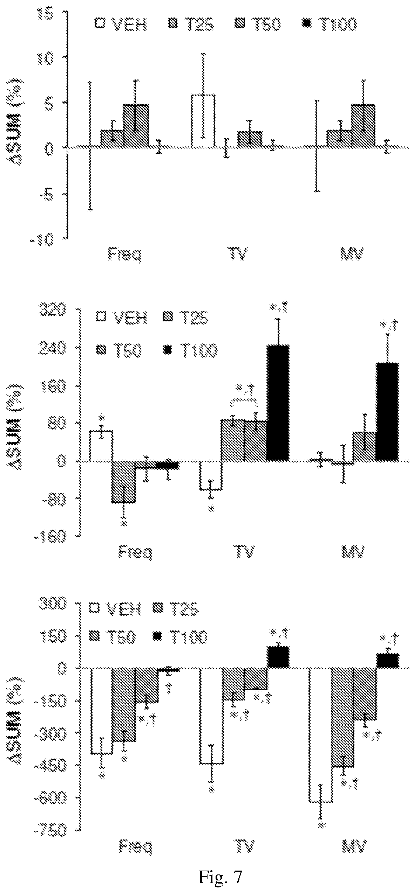

Fig. 7 illustrates a summary of the total changes in ventilatory parameters elicited by bolus injections of vehicle (VEH) or Tempol (25, 50 or 100 mg/kg, IV; T25, T50, T100, respectively) and subsequent injection of fentanyl (5 µg/kg, IV) in isoflurane-anesthetized rats. There were 12 rats in the vehicle group, and 3, 5 and 12 rats in the 25, 50 and 100 mg/kg Tempol groups, respectively. Data are mean ± SEM. *P < 0.05, significant change. ^{†}P < T25, T50 and/or T100 versus vehicle.

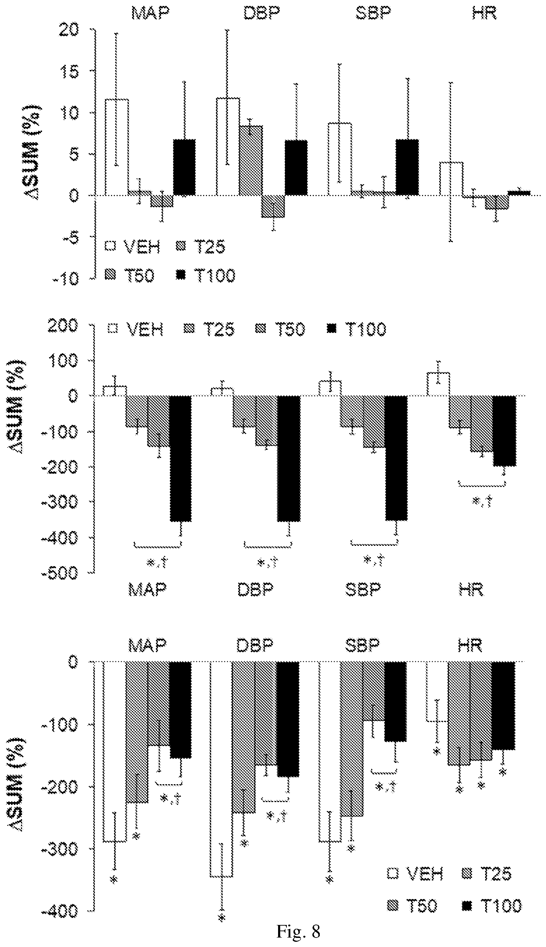

Fig. 8 illustrates a summary of the total changes in mean (MAP), diastolic (DBP) and systolic (SBP) arterial blood pressures and heart rate elicited by bolus injections of vehicle (VEH) or Tempol (25, 50 or 100 mg/kg, IV; T25, T50, T100, respectively) and subsequent injection of fentanyl (5 µg/kg, IV) in isoflurane-anesthetized rats. There were 12 rats in the vehicle group, and 3, 5 and 12 rats in the 25, 50 and 100 mg/kg Tempol groups, respectively. The data are presented as mean ± SEM. *P < 0.05, significant change. ^{†}P < T25, T50 and/or T100 versus vehicle.

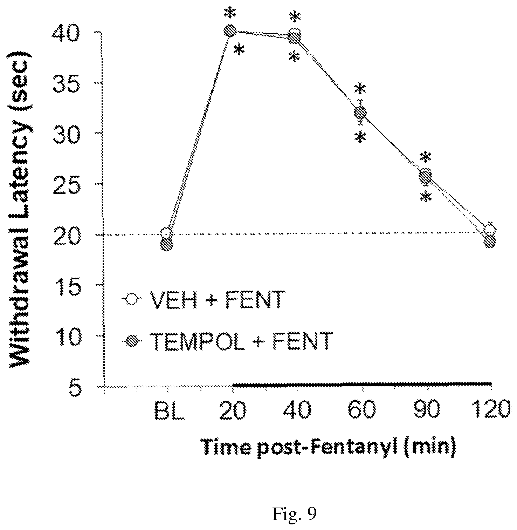

Fig. 9 illustrates a summary of the changes in tail-flick withdrawal latency from baseline (BL) elicited by a bolus injection of fentanyl (25 µg/kg, IV) in conscious rats that had received an injection of Vehicle or Tempol (100 mg/kg, IV) 20 minutes previously. There were 6 rats in each group. The data are presented as mean ± SEM. *P < 0.05, significant from baseline. There were no differences between Vehicle- or Tempol-treated rats at any time point (P > 0.05, for all comparisons).

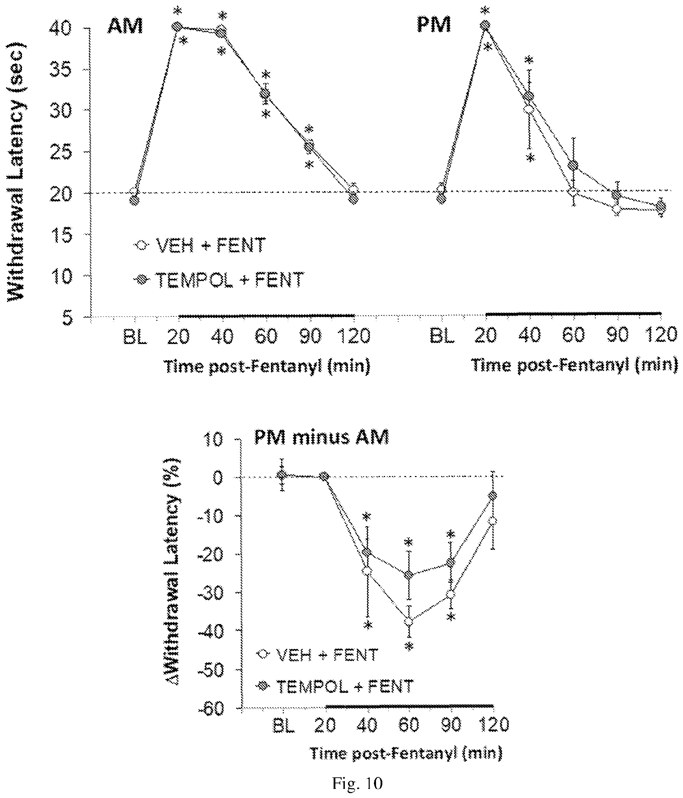

Fig. 10 illustrates a summary of the changes in tail-flick withdrawal latency from baseline (BL) elicited by a bolus injection of fentanyl (25 µg/kg, IV) in conscious rats that had received an injection of Vehicle (VEH) or Tempol (100 mg/kg, IV) 20 minutes previously. The top-left panel shows the data from the first injection protocol commenced at 10 AM. The top right panel shows the data from a second injection of fentanyl in the same rats and 4 PM (no vehicle or tempol pretreatment). The bottom panel shows arithmetic differences in tail-flick withdrawal latencies between the PM and AM studies. There were 6 rats in each group. The data are shown as mean ± SEM. *P < 0.05, significantly different from baseline. There were no differences between Vehicle- or Tempol-treated rats at any time point (P > 0.05, for all comparisons).

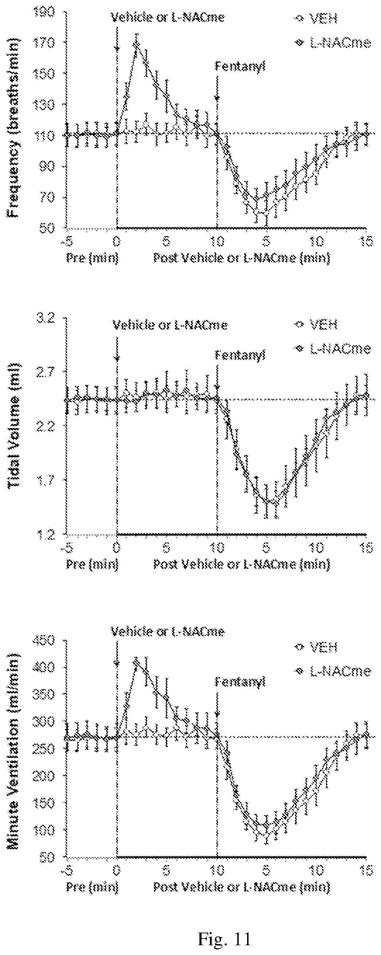

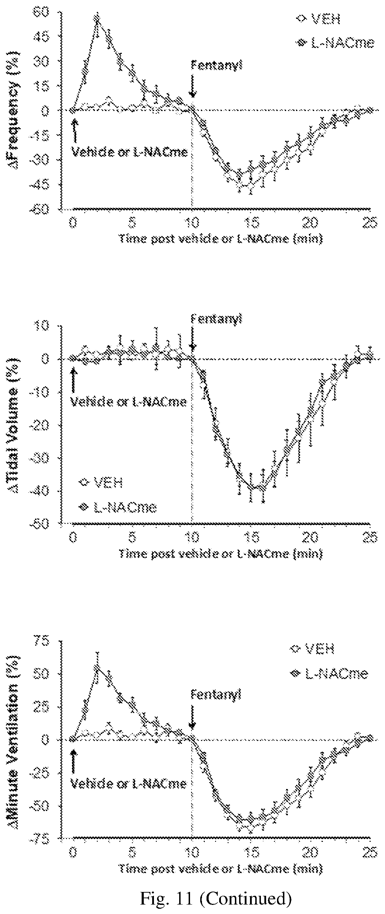

Fig. 11 illustrates a summary of the changes in frequency of breathing, tidal volume and minute ventilation elicited by bolus injections of vehicle or L-acetyl-N-cysteine (L-NACme, 500 µmol/kg, IV) and subsequent injections of fentanyl (25 µg/kg, IV) in freely-moving rats. There were 9 rats in each group. The data are presented as mean ± SEM. There were no differences between Vehicle- or Tempol-treated rats at any time point (P > 0.05, for all comparisons).

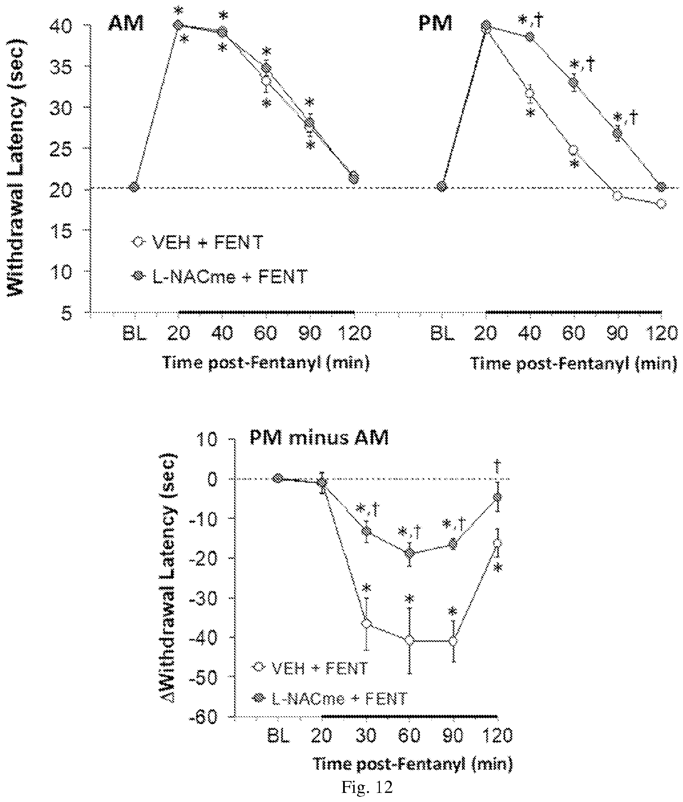

Fig. 12 illustrates a summary of the changes in tail-flick withdrawal latency from baseline (BL) elicited by a bolus injection of fentanyl (25 µg/kg, IV) in conscious rats that had received an injection of Vehicle or L-NACme (500 µmol/kg, IV) 20 minutes previously. The top-left panel shows the data from the first injection protocol commenced at 10 AM. The top right panel shows the data from a second injection of fentanyl in the same rats and 4 PM (no vehicle or L-NACme pretreatment). The bottom panel shows the arithmetic differences in the tail-flick withdrawal latencies between the PM and AM studies. There were 6 rats in each group. The data are shown as mean ± SEM. *P < 0.05, significantly different from baseline. ^{†}P < L-NACme + fentanyl versus vehicle + fentanyl.

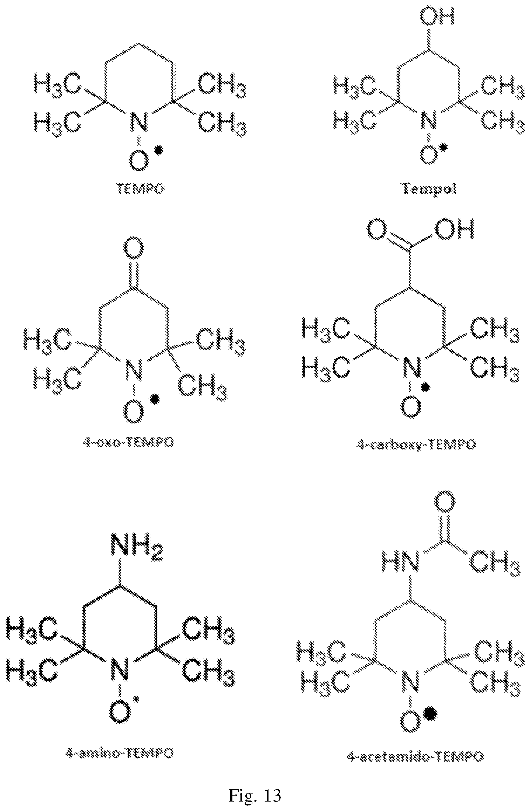

Fig. 13 illustrates some tempol and tempol-related structures.

### DETAILED DESCRIPTION

Methods involving conventional molecular biology techniques are described herein. Such techniques are generally known in the art and are described in detail in methodology treatises, such as Current Protocols in Molecular Biology, ed. Ausubel et al., Greene Publishing and Wiley-Interscience, New York, 1992 (with periodic updates). Unless otherwise defined, all technical terms used herein have the same meaning as commonly understood by one of ordinary skill in the art to which the present invention pertains. Commonly understood definitions of molecular biology terms can be found in, for example, Rieger et al., Glossary of Genetics: Classical and Molecular, 5th Edition, Springer-Verlag: New York, 1991, and Lewin, Genes V, Oxford University Press: New York, 1994. The definitions provided herein are to facilitate understanding of certain terms used frequently herein and are not meant to limit the scope of the present invention.

The articles "a" and "an" are used herein to refer to one or to more than one (*i.e.,* to at least one) of the grammatical object of the article. By way of example, "an element" means one element or more than one element.

The terms "comprise," "comprising," "include," "including," "have," and "having" are used in the inclusive, open sense, meaning that additional elements may be included. The terms "such as", "*e.g.,*"*,* as used herein are non-limiting and are for illustrative purposes only. "Including" and "including but not limited to" are used interchangeably.

The term "or" as used herein should be understood to mean "and/or" unless the context clearly indicates otherwise.

The term "about" or "approximately" as used herein refers to a quantity, level, value, number, frequency, percentage, dimension, size, amount, weight or length that varies by as much as 15%, 10%, 9%, 8%, 7%, 6%, 5%, 4%, 3%, 2% or 1% to a reference quantity, level, value, number, frequency, percentage, dimension, size, amount, weight or length. In one embodiment, the term "about" or "approximately" refers a range of quantity, level, value, number, frequency, percentage, dimension, size, amount, weight or length ± 15%, ± 10%, ± 9%, ± 8%, ± 7%, ± 6%, ± 5%, ± 4%, ± 3%, ± 2%, or ± 1% about a reference quantity, level, value, number, frequency, percentage, dimension, size, amount, weight or length.

The phrases "parenteral administration" and "administered parenterally" are art-recognized terms, and include modes of administration other than enteral and topical administration, such as injections, and include, without limitation, intravenous, intramuscular, intrapleural, intravascular, intrapericardial, intraarterial, intrathecal, intracapsular, intraorbital, intracardiac, intradermal, intraperitoneal, transtracheal, subcutaneous, subcuticular, intra-articular, subcapsular, subarachnoid, intraspinal and intrastemal injection and infusion.

The term "treating" is art-recognized and includes inhibiting a disease, disorder or condition in a subject, *e.g*., impeding its progress; and relieving the disease, disorder or condition, *e.g*., causing regression of the disease, disorder and/or condition. Treating the disease or condition includes ameliorating at least one symptom of the particular disease or condition, even if the underlying pathophysiology is not affected.

The term "preventing" is art-recognized and includes stopping a disease, disorder or condition from occurring in a subject, which may be predisposed to the disease, disorder and/or condition but has not yet been diagnosed as having it. Preventing a condition related to a disease includes stopping the condition from occurring after the disease has been diagnosed but before the condition has been diagnosed.

The term "pharmaceutical composition" refers to a formulation containing the disclosed compounds in a form suitable for administration to a subject. In some embodiments, the pharmaceutical composition is in bulk or in unit dosage form. The unit dosage form is any of a variety of forms, including, for example, a capsule, an IV bag, a tablet, a single pump on an aerosol inhaler, or a vial. The quantity of active ingredient (*e.g*., a formulation of the disclosed compound or salts thereof) in a unit dose of composition is an effective amount and is varied according to the particular treatment involved. One skilled in the art will appreciate that it is sometimes necessary to make routine variations to the dosage depending on the age and condition of the patient. The dosage will also depend on the route of administration. A variety of routes are contemplated, including oral, pulmonary, rectal, parenteral, transdermal, subcutaneous, intravenous, intramuscular, intraperitoneal, intranasal, inhalational, and the like. Dosage forms for the topical or transdermal administration of a compound described herein includes powders, sprays, ointments, pastes, creams, lotions, gels, solutions, patches, nebulized compounds, and inhalants. In some embodiments, the compound or active ingredient is mixed under sterile conditions with a pharmaceutically acceptable carrier, and with any preservatives, buffers, or propellants that are required.

The phrase "pharmaceutically acceptable" is art-recognized. In certain embodiments, the term includes compositions, polymers and other materials and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

The phrase "pharmaceutically acceptable carrier" is art-recognized, and includes, for example, pharmaceutically acceptable materials, compositions or vehicles, such as a liquid or solid filler, diluent, excipient, solvent or encapsulating material, involved in carrying or transporting any subject composition from one organ, or portion of the body, to another organ, or portion of the body. Each carrier must be "acceptable" in the sense of being compatible with the other ingredients of a subject composition and not injurious to the patient. In certain embodiments, a pharmaceutically acceptable carrier is non-pyrogenic. Some examples of materials, which may serve as pharmaceutically acceptable carriers include: (1) sugars, such as lactose, glucose and sucrose; (2) starches, such as corn starch and potato starch; (3) cellulose, and its derivatives, such as sodium carboxymethyl cellulose, ethyl cellulose and cellulose acetate; (4) powdered tragacanth; (5) malt; (6) gelatin; (7) talc; (8) excipients, such as cocoa butter and suppository waxes; (9) oils, such as peanut oil, cottonseed oil, sunflower oil, sesame oil, olive oil, corn oil and soybean oil; (10) glycols, such as propylene glycol; (11) polyols, such as glycerin, sorbitol, mannitol and polyethylene glycol; (12) esters, such as ethyl oleate and ethyl laurate; (13) agar; (14) buffering agents, such as magnesium hydroxide and aluminum hydroxide; (15) alginic acid; (16) pyrogen-free water; (17) isotonic saline; (18) Ringer's solution; (19) ethyl alcohol; (20) phosphate buffer solutions; and (21) other non-toxic compatible substances employed in pharmaceutical formulations.

The compounds of the application are capable of further forming salts. All of these forms are also contemplated herein.

"Pharmaceutically acceptable salt" of a compound means a salt that is pharmaceutically acceptable and that possesses the desired pharmacological activity of the parent compound. For example, the salt can be an acid addition salt. One embodiment of an acid addition salt is a hydrochloride salt. The pharmaceutically acceptable salts can be synthesized from a parent compound that contains a basic or acidic moiety by conventional chemical methods. Generally, such salts can be prepared by reacting the free acid or base forms of these compounds with a stoichiometric amount of the appropriate base or acid in water or in an organic solvent, or in a mixture of the two; generally, non-aqueous media like ether, ethyl acetate, ethanol, isopropanol, or acetonitrile being preferred. Lists of salts are found in Remington's Pharmaceutical Sciences, 18th ed. (Mack Publishing Company, 1990).

The compounds described herein can also be prepared as esters, for example pharmaceutically acceptable esters. For example, a carboxylic acid function group in a compound can be converted to its corresponding ester, *e*.*g*., a methyl, ethyl, or other ester. Also, an alcohol group in a compound can be converted to its corresponding ester, *e.g.,* an acetate, propionate, or other ester.

The compounds described herein can also be prepared as prodrugs, for example pharmaceutically acceptable prodrugs. The terms "pro-drug" and "prodrug" are used interchangeably herein and refer to any compound, which releases an active parent drug *in vivo.* Since prodrugs are known to enhance numerous desirable qualities of pharmaceuticals (*e.g*., solubility, bioavailability, manufacturing, etc.) the compounds can be delivered in prodrug form. Thus, the compounds described herein are intended to cover prodrugs of the presently claimed compounds, methods of delivering the same and compositions containing the same. "Prodrugs" are intended to include any covalently bonded carriers that release an active parent drug *in vivo* when such prodrug is administered to a subject. Prodrugs are prepared by modifying functional groups present in the compound in such a way that the modifications are cleaved, either in routine manipulation or *in vivo,* to the parent compound. Prodrugs include compounds wherein a hydroxy, amino, sulfhydryl, carboxy, or carbonyl group is bonded to any group that may be cleaved *in vivo* to form a free hydroxyl, free amino, free sulfhydryl, free carboxy or free carbonyl group, respectively. Prodrugs can also include a precursor (forerunner) of a compound described herein that undergoes chemical conversion by metabolic processes before becoming an active or more active pharmacological agent or active compound described herein.

Additionally, the salts of the compounds described herein, can exist in either hydrated or unhydrated (the anhydrous) form or as solvates with other solvent molecules. Nonlimiting examples of hydrates include monohydrates, dihydrates, etc. Nonlimiting examples of solvates include ethanol solvates, acetone solvates, etc.

The term "solvates" means solvent addition forms that contain either stoichiometric or non stoichiometric amounts of solvent. Some compounds have a tendency to trap a fixed molar ratio of solvent molecules in the crystalline solid state, thus forming a solvate. If the solvent is water the solvate formed is a hydrate, when the solvent is alcohol, the solvate formed is an alcoholate. Hydrates are formed by the combination of one or more molecules of water with one of the substances in which the water retains its molecular state as H₂O, such combination being able to form one or more hydrate.

The compounds, salts and prodrugs described herein can exist in several tautomeric forms, including the enol and imine form, and the keto and enamine form and geometric isomers and mixtures thereof. Tautomers exist as mixtures of a tautomeric set in solution. In solid form, usually one tautomer predominates. Even though one tautomer may be described, the present application includes all tautomers of the present compounds. A tautomer is one of two or more structural isomers that exist in equilibrium and are readily converted from one isomeric form to another. This reaction results in the formal migration of a hydrogen atom accompanied by a switch of adjacent conjugated double bonds. In solutions where tautomerization is possible, a chemical equilibrium of the tautomers will be reached. The exact ratio of the tautomers depends on several factors, including temperature, solvent, and pH. The concept of tautomers that are interconvertable by tautomerizations is called tautomerism.

A "patient," "subject," or "host" to be treated by the compounds or methods described herein may mean either a human or non-human animal, such as a mammal, a fish, a bird, a reptile, or an amphibian. Thus, the subject of the herein disclosed methods can be a human, non-human primate, horse, pig, rabbit, dog, sheep, goat, cow, cat, guinea pig or rodent. The term does not denote a particular age or sex. Thus, adult and newborn subjects, as well as fetuses, whether male or female, are intended to be covered. In one aspect, the subject is a mammal. A patient refers to a subject afflicted with a disease or disorder.

The terms "prophylactic" or "therapeutic" treatment is art-recognized and includes administration to the host of one or more of the subject compounds. If it is administered prior to clinical manifestation of the unwanted condition (*e.g*., disease or other unwanted state of the host animal) then the treatment is prophylactic, *i.e.,* it protects the host against developing the unwanted condition, whereas if it is administered after manifestation of the unwanted condition, the treatment is therapeutic (*i.e.,* it is intended to diminish, ameliorate, or stabilize the existing unwanted condition or side effects thereof).

The terms "therapeutic agent", "drug", "medicament", "active ingredient", and "bioactive substance" are art-recognized and include molecules and other agents that are biologically, physiologically, or pharmacologically active substances that act locally or systemically in a patient or subject to treat a disease or condition. The terms include without limitation pharmaceutically acceptable salts thereof and prodrugs. Such agents may be acidic, basic, or salts; they may be neutral molecules, polar molecules, or molecular complexes capable of hydrogen bonding; they may be prodrugs in the form of ethers, esters, amides and the like that are biologically activated when administered into a patient or subject.

The phrase "therapeutically effective amount" or "pharmaceutically effective amount" is an art-recognized term. In certain embodiments, the term refers to an amount of a therapeutic agent that produces some desired effect at a reasonable benefit/risk ratio applicable to any medical treatment. In certain embodiments, the term refers to that amount necessary or sufficient to eliminate, reduce or maintain a target of a particular therapeutic regimen. The effective amount may vary depending on such factors as the disease or condition being treated, the particular targeted constructs being administered, the size of the subject or the severity of the disease or condition. One of ordinary skill in the art may empirically determine the effective amount of a particular compound without necessitating undue experimentation. In certain embodiments, a therapeutically effective amount of a therapeutic agent for *in vivo* use will likely depend on a number of factors, including: the rate of release of an agent from a polymer matrix, which will depend in part on the chemical and physical characteristics of the polymer; the identity of the agent; the mode and method of administration; and any other materials incorporated in the polymer matrix in addition to the agent.

Throughout the description, where compositions are described as having, including, or comprising, specific components, it is contemplated that compositions also consist essentially of, or consist of, the recited components. Similarly, where methods or processes are described as having, including, or comprising specific process steps, the processes also consist essentially of, or consist of, the recited processing steps. Further, it should be understood that the order of steps or order for performing certain actions is immaterial so long as the compositions and methods described herein remains operable. Moreover, two or more steps or actions can be conducted simultaneously.

All percentages and ratios used herein, unless otherwise indicated, are by weight.

Embodiments described herein relate to a composition comprising a nitroxide compound for use in the treatment of opioid induced respiratory depression in a subject in need thereof, by administering to said subject a therapeutically effective amount of said composition comprising a nitroxide compound, wherein the nitroxide compound comprises at least one of 4-hydroxy-2,2,6,6-tetramethylpiperidine-1-oxyl (Tempol), 2,2,6,6-tetramethylpiperidine-1-oxyl (Tempo), 4-amino-2,2,6,6-tetramethyl-1-piperidinyloxy (Tempamine); 4-oxo-2,2,6,6-tetramethyl-1-piperidinyloxy (4-oxo-Tempo), 4-carboxy-2,2,6,6-tetramethyl-1-piperidinyloxy (4-carboxy-Tempo), 4-acetamido-2,2,6,6-tetramethyl-1-piperidinyloxy, 3-aminomethyl- 2,2,5,5-tetramethyl-1 -pyrrolidinyl-N-oxyl (3-aminomethyl-Proxyl), 3-cyano-2,2,5,5- tetramethyl-1-pyrrolidinyl-N-oxyl (3-cyano-Proxyl), 3-Carbamoyl-2,2,5,5-tetramethyl-1- pyrrolidinyl-N-oxyl (3-Carbamoyl-Proxyl), 3-Carboxy-2,2,5,5-tetramethyl-1-pyrrolidinyl-N- oxyl (3-Carboxy-Proxyl),or pharmaceutically acceptable salts, tautomers, or solvates thereof.

Nitroxides are stable free radicals that are shown to have antioxidant catalytic activities, which mimic those of superoxide dismutase (SOD), and which when existing in vivo, can interact with other substances. It was found that administration of nitroxide compounds, such as Tempol, dose-dependently and markedly attenuated the cardio and/or respiratory depressant effects elicited by opioids, such as fentanyl, without affecting opioid-induced analgesia.

In some embodiments, the treatment of opioid induced respiratory depression in a subject in need thereof is attenuation of opioid induced respiratory depression in a subject in need thereof, and the therapeutically effective amount of said composition comprising a nitroxide compound is an amount effective to attenuate the opioid induced respiratory depression.

The amount or therapeutically effective amount of the nitroxide compound that is administered to the subject can be an amount effective to increase tidal volume, increase respiratory frequency, increase minute ventilation, increase mean arterial blood pressure, increase diastolic blood pressure, and/or increase systolic blood pressure in the subject. For example, an opioid upon administration to a subject can depress at least one tidal volume, respiratory frequency, minute ventilation, mean arterial blood pressure, diastolic blood pressure, or systolic blood pressure at least about 1%, at least about 2%, at least about 3%, at least about 4%, at least about 5%, at least about 6%, at least about 7%, at least about 8%, at least about 9%, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80% or more, and the nitroxide compound can be administered to the subject at an amount effective to increase at least one of the opioid depressed tidal volume, respiratory frequency, minute ventilation, mean arterial blood pressure, diastolic blood pressure, or systolic blood pressure at least about 1%, at least about 2%, at least about 3%, at least about 4%, at least about 5%, at least about 6%, at least about 7%, at least about 8%, at least about 9%, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80% or more.

In the nitroxide compounds described herein, the unpaired electron is stable in part because the nitrogen nucleus is attached to two carbon atoms which are substituted with strong electron donors. With the partial negative charge on the oxygen of the N-O bond, the two adjacent carbon atoms together localize the unpaired electron on the nitrogen nucleus.

The nitroxide compounds described herein have a heterocyclic structure. The fundamental criterion is a stable free radical. In such heterocyclic structures, stable isotopes may be utilized (*e.g*., deuterium).

Composition comprising a nitroxide compound described herein can be administered to a subject to attenuate opioid induced respiratory depression in the subject in need thereof. The opioid, which induces respiratory depression in the subject, can include, but is not limited to, alfentanil, buprenorphine, butorphanol, carfentanil, codeine, diamorphine, dextromoramide, dezocine, dihydrocodeine, fentanyl, hydrocodone, hydromorphone, levorphanol, meperidine, meptazinol, methadone, morphine, nalbuphine, nalorphine, opium, oxycodone, oxymorphone, pentazocine, propoxyphene, remifentanil, sufentanil, tapentadol, and tramadol, and pharmaceutically acceptable salts thereof. For example, the opioid can include carfentanil, fentanyl, remifentanil, or sufentanil.

In some embodiments, the composition including the nitroxide compound can be administered to the subject to prevent the need for mechanical ventilation in subjects with acutely impaired ventilatory and/or respiratory drive because of an acute exacerbation of an underlying lung disease or an acute requirement for narcotic analgesia. For example, the subjects can be at-risk subjects with severe, hypercapneic COPD or mixed apnea evident on polysomnography.

In other embodiments, the subject can have or has an increased risk of cardio and/or respiratory depression that is caused, for example, by an anesthetic, a sedative, anxiolytic agent, a hypnotic agent, alcohol, and/or a narcotic. By way of a non-limiting example, narcotic analgesics (*e.g*., morphine, fentanyl, oxycodone, buprenorphine) are administered to cancer patients to alleviate pain. The dose is often limited by a fear of respiratory depression. In addition, even a partial respiratory depression from these drugs causes hypoxia and a resulting excessive daytime sleepiness that can be debilitating and severely decrease quality of life. General anesthetics can exert a similar depressant effect on respiration and delay a patient's transfer from the operating room to a surgical recovery area. A composition comprising a nitroxide compound described herein is therefore useful to counteract the lingering effects of the anesthetic, and for restoring adequate respiratory drive to enable the patient to breathe on their own.

In other embodiments, a composition including a nitroxide compound described herein can be administered in ambulatory delivery formulations to treat respiratory depression associated with narcotics, analgesics, sedatives, and/or opioids. The subject can be one who is taking and/or over-dosed on the narcotics, analgesics, sedatives, and/or opioids and who is experiencing or at risk of acute cardio and/or respiratory depression. The compositions can be administered to the subject to increase at least one of opioid depressed tidal volume, respiratory frequency, minute ventilation, mean arterial blood pressure, diastolic blood pressure, or systolic blood pressure.

In some embodiments, a subject can include a subject with an increased risk of decreased cardio and/or respiratory drive such as a subject with a significant chronic obstructive pulmonary disease, and those with a substantially decreased respiratory reserve, hypoxia, hypercapnia, or pre-existing respiratory depression. Elderly, cachectic, or debilitated subjects may have altered pharmacokinetics or altered opioid clearance compared to younger, healthier patients resulting in greater risk for respiratory depression.

In some embodiments, compositions including a nitroxide compound described herein herein can be administered to the subject in combination with at least one additional compound, agent, and/or therapeutic agent useful for treating the subject or the breathing disorder. These additional compounds, agents, and/or therapeutic agents can include commercially available agents or compounds, known to treat, prevent, or reduce the symptoms of breathing disorders or treat the disorder in the subject.

In some embodiments, the at least one additional therapeutic agent can change normal breathing in a subject. Such additional agents can be selected from the group consisting of an opioid, doxapram and enantiomers thereof, acetazolamide, almitrine, theophylline, caffeine, methylprogesterone and related compounds, sedatives that decrease arousal threshold in sleep disordered breathing patients, sodium oxybate, benzodiazepine receptor agonists, orexin antagonists, tricyclic antidepressants, serotonergic modulators, adenosine and adenosine receptor and nucleoside transporter modulators, cannabinoids, orexins, melatonin agonists, ampakines, and combinations thereof.

In other embodiments, compositions comprising a nitroxide compound described herein and at least one additional compound has additive, complementary or synergistic effects in the treatment of the breathing disorder or other disorder in the subject. In a non-limiting example, the compositions that include a nitroxide compound described herein may be used concurrently or in combination with one or more of the following drugs: an opioid (*e.g*., morphine, oxycodone, fentanyl), doxapram, enantiomers of doxapram, acetazolamide, almitrine, theophylline, caffeine, methylprogesterone and related compounds, sedatives that decrease arousal threshold in sleep disordered breathing patients (*e.g*., eszopiclone and zolpidem), sodium oxybate, benzodiazepine receptor agonists (*e.g*., zolpidem, zaleplon, eszopiclone, estazolam, flurazepam, quazepam, temazepam, triazolam), orexin antagonists *(e.g.,* suvorexant), tricyclic antidepressants *(e.g.,* doxepin), serotonergic modulators, adenosine and adenosine receptor and nucleoside transporter modulators, cannabinoids (*e.g.,* but not limited to, dronabinol), orexins, melatonin agonists *(e.g.,* ramelteon) and compounds known as ampakines.

The combination of two or more compounds may refer to a composition wherein the individual compounds are physically mixed or wherein the individual compounds are physically separated. A combination therapy encompasses administering the components separately to produce the desired additive, complementary or synergistic effects.

In one embodiment, the composition comprising a nitroxide compound described herein and an additional agent are physically mixed in the composition. In another embodiment, the composition comprising a nitroxide compound described herein and the additional agent are physically separated in the composition.

In one embodiment, compositions including a nitroxide compound described herein are co-administered with a compound that is used to treat another disorder but causes loss of breathing control. In this aspect, compositions including a nitroxide compound described herein block or otherwise reduce depressive effects on normal breathing control caused by the compound with which they are co-administered. An exemplary compound that treats another disorder but depresses breathing control includes but is not limited to anesthetics, sedatives, sleeping aids, anxiolytics, hypnotics, alcohol, and narcotic analgesics. The co-administered compound may be administered individually, or a combined composition as a mixture of solids and/or liquids in a solid, gel or liquid formulation or as a solution, according to methods known to those familiar with the art.

In some embodiments, a composition including a nitroxide compound described herein may be packaged with at least one additional compound useful for treating breathing control disorders. In another embodiment, a composition including a nitroxide compound described herein may be packaged with a therapeutic agent known to cause changes in breathing control, such as, but not limited to, anesthetics, sedatives, anxiolytics, hypnotics, alcohol, and narcotic analgesics. A co-package may be based upon, but not limited to, dosage units. For example, a composition can include an opioid capable of inducing cardio and/or respiratory depression in a subject and an amount of a nitroxide compound described herein effective to prevent the opioid induced cardio and/or respiratory depression when the composition is administered to the subject.

In some embodiments, an effective amount (*i.e*., dose) of a nitroxide compound described herein (*e.g*., Tempol) to be administered to a subject can be determined depending upon, for example, age, body weight, symptom, the desired therapeutic effect, the route of administration, and the duration of the treatment. Exemplary doses can be from about 0.01 to about 1000 mg, by oral administration. Examples of dose ranges can include from a minimum dose of about 0.01, 0.10, 0.50, 1, 5, 10, 25, 50, 100, 125, 150, 200, or 250 mg to a maximum dose of about 300, 400, 500, 600, 700, 800, 900, or 1000 mg, wherein the dose range can include from any one of the foregoing minimum doses to any one of the foregoing maximum doses. Specific examples of particular effective amounts contemplated via oral administration can include about 0.02, 0.03, 0.04, 0.05, 0.10, 0.15, 0.20, 0.25, 0.30, 0.35, 0.40, 0.45, 0.50, 0.55, 0.60, 0.65, 0.70, 0.75, 0.80, 0.85, 0.90, 0.95, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 105, 110, 115, 120, 125, 130, 135, 140, 145, 150, 155, 160, 165, 170, 175, 180, 185, 190, 195, 200, 205, 210, 215, 220, 225, 230, 235, 240, 245, 250, 255, 260, 265, 270, 275, 280, 285, 290, 295, 300, 305, 310, 315, 320, 325, 330, 335, 340, 345, 350, 355, 360, 365, 370, 375, 380, 385, 390, 395, 400, 405, 410, 415, 420, 425, 430, 435, 440, 445, 450, 455, 460, 465, 470, 475, 480, 485, 490, 495, 500, 505, 510, 515, 520, 525, 530, 535, 540, 545, 550, 555, 560, 565, 570, 575, 580, 585, 590, 595, 600, 605, 610, 615, 620, 625, 630, 635, 640, 645, 650, 655, 660, 665, 670, 675, 680, 685, 690, 695, 700, 705, 710, 715, 720, 725, 730, 735, 740, 745, 750, 755, 760, 765, 770, 775, 780, 785, 790, 795, 800, 805, 810, 820, 825, 830, 835, 840, 845, 850, 855, 860, 865, 870, 875, 880, 885, 890, 895, 900, 905, 910, 915, 920, 925, 930, 935, 940, 945, 950, 955, 960, 965, 970, 975, 980, 985, 990, 995, 1000 mg or more. The oral dose can be administered once daily, twice daily, three times daily, or more frequently.

The dose of the nitroxide compound (*e.g*., Tempol) for use in parenteral administration (*e.g*., intravenous administration) is generally from about 0.01 to about 300 mg/kg body weight. Examples of dose ranges can include from a minimum dose of about 0.01, 0.10, 0.50, 1, 5, 10, 25, 50, or 100 mg/kg body weight to a maximum dose of about 125, 150, 175, 200, 250, 275, or 300 mg/kg body weight, wherein the dose range can include from any one of the foregoing minimum doses to any one of the foregoing maximum doses. Specific examples of effective amounts contemplated include about 0.02, 0.03, 0.04, 0.05, 0.10, 0.15, 0.20, 0.25, 0.30, 0.35, 0.40, 0.45, 0.50, 0.55, 0.60, 0.65, 0.70, 0.75, 0.80, 0.85, 0.90, 0.95, 1, 2, 3, 4, 5, 6, 7, 8, 9, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 195, 200, 205, 210, 215, 220, 225, 230, 235, 240, 245, 250, 255, 260, 265, 270, 275, 280, 285, 290, 295, 300 mg/kg body weight or more. Continuous intravenous administration is also contemplated for from 1 to 24 hours per day to achieve a target concentration from about 0.01 mg/L blood to about 100 mg/L blood. Exemplary dose ranges can include from a minimum dose of about 0.01, 0.10, 0.25, 0.50, 1, 5, 10, or 25 mg/L blood to a maximum dose of about 40, 45, 50, 55, 60, 65, 70, 75, 80, 85, 90, or 100 mg/L, wherein an exemplary dose ranges can include from any one of the foregoing minimum doses to any one of the foregoing maximum doses. Specific examples of particular effective amounts contemplated via this route include about 0.02, 0.03, 0.04, 0.05, 0.10, 0.15, 0.20, 0.25, 0.30, 0.35, 0.40, 0.45, 0.50, 0.55, 0.60, 0.65, 0.70, 0.75, 0.80, 0.85, 0.90, 0.95, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100 mg/L blood or more. The dose to be used can depend upon various conditions, and there may be cases wherein doses lower than or greater than the ranges specified above are used.

The nitroxide compounds described herein (*e*.*g*., Tempol) may be administered in the form of, for example, solid compositions, liquid compositions, or other compositions for oral administration, injections, liniments, or suppositories for parenteral administration. Solid compositions for oral administration include compressed tablets, pills, capsules, dispersible powders, and granules. Capsules include hard capsules and soft capsules. In such solid compositions, the stable nitroxide can be admixed with an excipient (e.g., lactose, mannitol, glucose, microcrystalline cellulose, or starch), combining agents (*e.g*., hydroxypropyl cellulose, polyvinyl pyrrolidone, or magnesium metasilicate aluminate), disintegrating agents *(e.g.,* cellulose calcium glycolate), lubricating agents *(e.g.,* magnesium stearate), stabilizing agents, agents to assist dissolution (*e.g*., glutamic acid or aspartic acid), or the like. The agents may, if desired, be coated with coating agents (*e.g*., sugar, gelatin, hydroxypropyl cellulose, or hydroxypropylmethyl cellulose phthalate), or be coated with two or more films. Further, coating may include containment within capsules of absorbable materials such as gelatin.

Liquid compositions for oral administration include pharmaceutically acceptable solutions, suspensions, emulsions, syrups, and elixirs. In such compositions, the nitroxide compound is dissolved, suspended, or emulsified in a commonly used diluent (*e.g*., purified water, ethanol, or mixture thereof). Furthermore, such liquid compositions may also comprise wetting agents, suspending agents, emulsifying agents, flavoring agents (*e.g*., flavor-masking agents) sweetening agents, perfuming agents, preserving agents, buffer agents, or the like.

Injections for parenteral administration include solutions, suspensions, emulsions, and solids, which are dissolved or suspended. For injections, the nitroxide compound can be dissolved, suspended, and/or emulsified in a solvent. The solvents are, for example, distilled water for injection, physiological salt solution, vegetable oil, propylene glycol, polyethylene glycol, alcohol such as ethanol, or a mixture thereof. Moreover the injections also can include stabilizing agents, agents to assist dissolution (*e.g*., glutamic acid, aspartic acid, or POLYSORBATE 80), suspending agents, emulsifying agents, soothing agents, buffer agents, preserving agents, etc. The compositions are sterilized in the final process or manufactured and prepared by sterile procedure. The compositions also can be manufactured in the form of sterile solid compositions, such as a freeze-dried composition, and can be sterilized or dissolved immediately before use in sterile distilled water for injection or some other solvent.

Other compositions for parenteral administration include liquids and ointments for external use, endermic liniments, compositions for inhalation, sprays, suppositories for rectal administration, and pessaries for vaginal administration, which compositions include a stable nitroxide and are administered by methods known in the art.

Nitroxide compositions for inhalation or sprays may comprise additional substances other than diluents, such as, *e.g.,* stabilizing agents *(e.g.,* sodium sulfite hydride), isotonic buffers (*e.g.*, sodium chloride, sodium citrate or citric acid). See, for example, the methods described in U.S. Pat. Nos. 2,868,691 and 3,095,355. The nitroxide compound can be effectively distributed by inhalation or spray using a self-propelling composition that includes a solution or dispersion of the stable nitroxide in micronized form. For example, an effective dispersion of finely divided drug particles can be accomplished with the use of very small quantities of a suspending agent, present as a coating on micronized drug particles. Evaporation of the propellant from the aerosol particles after spraying from the aerosol container leaves finely divided drug particles coated with a fine film of the suspending agent. In the micronized form, the average particle size can be less than about 5 microns. The propellant composition can employ, as the suspending agent, a fatty alcohol such as oleyl alcohol. Propellants that may be employed include hydrofluoroalkane propellants and chlorofluorocarbon propellants. Dry powder inhalation also can be employed.

The route(s) of administration will be readily apparent to the skilled artisan and will depend upon any number of factors including the type and severity of the disease being treated, the type and age of the veterinary or human patient being treated, and the like.

The formulations of the pharmaceutical compositions described herein may be prepared by any method known or hereafter developed in the art of pharmacology and pharmaceutics. In general, such preparatory methods include the step of bringing the active ingredient into association with a carrier or one or more other accessory ingredients, and then, if necessary or desirable, shaping or packaging the product into a desired single-dose or multi-dose unit.

Although the descriptions of pharmaceutical compositions provided herein are principally directed to pharmaceutical compositions, which are suitable for ethical administration to humans, it will be understood by the skilled artisan that such compositions are generally suitable for administration to animals of all sorts. Modification of pharmaceutical compositions suitable for administration to humans in order to render the compositions suitable for administration to various animals is well understood, and the ordinarily skilled veterinary pharmacologist can design and perform such modification with merely ordinary, if any, experimentation. Subjects to which administration of the pharmaceutical compositions is contemplated include, but are not limited to, humans and other primates, mammals including commercially relevant mammals such as cattle, pigs, horses, sheep, cats, and dogs.

The regimen of administration may affect what constitutes an effective amount. The therapeutic formulations may be administered to the patient either prior to or after the onset of a breathing disorder event or ventilator depressant effects of the opioid. Further, several divided dosages, as well as staggered dosages may be administered daily or sequentially, or the dose may be continuously infused, or may be a bolus injection. Further, the dosages of the therapeutic formulations may be proportionally increased or decreased as indicated by the exigencies of the therapeutic or prophylactic situation.

Actual dosage levels of the active ingredients in the pharmaceutical compositions may be varied so as to obtain an amount of the active ingredient that is effective to achieve the desired therapeutic response for a particular patient, composition, and mode of administration, without being toxic to the patient.

A medical doctor, *e.g*., physician or veterinarian, having ordinary skill in the art may readily determine and prescribe the effective amount of the pharmaceutical composition required. For example, the physician or veterinarian could start doses of the compounds employed in the pharmaceutical composition at levels lower than that required in order to achieve the desired therapeutic effect and gradually increase the dosage until the desired effect is achieved.

Other embodiments described herein relate to a method of treating a subject in need thereof, such as a subject without normal ventilation and/or normal breathing control, by administering the compositions comprising a nitroxide compound described herein, and additionally treating the patient using a device to support breathing. Such devices include, but are not limited to, ventilation devices, CPAP and BiPAP devices.

Mechanical ventilation is a method to mechanically assist or replace spontaneous breathing. Mechanical ventilation is typically used after an invasive intubation, a procedure wherein an endotracheal or tracheostomy tube is inserted into the airway. It is normally used in acute settings, such as in the ICU, for a short period of time during a serious illness. It may also be used at home or in a nursing or rehabilitation institution, if patients have chronic illnesses that require long-term ventilation assistance. The main form of mechanical ventilation is positive pressure ventilation, which works by increasing the pressure in the patient's airway and thus forcing air into the lungs. Less common today are negative pressure ventilators (for example, the "iron lung") that create a negative pressure environment around the patient's chest, thus sucking air into the lungs. Types of mechanical ventilation are: conventional positive pressure ventilation, high frequency ventilation, non-invasive ventilation (non-invasive positive pressure ventilation or NIPPV), proportional assist ventilation (PAV), adaptive servo ventilation (ASV) and neurally adjusted ventilatory assist (NAVA).

Non-invasive ventilation refers to all modalities that assist ventilation without the use of an endotracheal tube. Non-invasive ventilation is primarily aimed at minimizing patient discomfort and the complications associated with invasive ventilation, and is often used in cardiac disease, exacerbations of chronic pulmonary disease, sleep apnea, and neuromuscular diseases. Non-invasive ventilation refers only to the patient interface and not the mode of ventilation used; modes may include spontaneous or control modes and may be either pressure or volume cycled modes.

Some commonly used modes of NIPPV include continuous positive airway pressure (CPAP). This kind of machine has been used mainly by patients for the treatment of sleep apnea at home, but now is in widespread use across intensive care units as a form of ventilatory support. The CPAP machine stops upper airway obstruction by delivering a stream of compressed air via a hose to a nasal pillow, nose mask or full-face mask, splinting the airway open (keeping it open under air pressure) so that unobstructed breathing becomes possible, reducing and/or preventing apneas and hypopneas. When the machine is turned on, but prior to the mask being placed on the head, a flow of air comes through the mask. After the mask is placed on the head, it is sealed to the face and the air stops flowing. At this point, it is only the air pressure that accomplishes the desired result. This has the additional benefit of reducing or eliminating the extremely loud snoring that sometimes accompanies sleep apnea.

Bi-level positive airway pressure (BIPAP) alternate between inspiratory positive airway pressure (IPAP) and a lower expiratory positive airway pressure (EPAP), triggered by patient effort. On many such devices, backup rates may be set, which deliver IPAP pressures even if patients fail to initiate a breath.

The invention is further illustrated by the following example, which is not intended to limit the scope of the claims.

### Example

There is conflicting evidence as to whether opioids induce oxidative stress. For example, there is compelling evidence that morphine, buprenorphine and methadone can both increase or decrease oxidative stress depending on the circumstances and experimental conditions. In contrast, the available evidence suggested that fentanyl and analogues either reduces or have no effect on oxidative stress. In order, to address the importance of superoxide anion in the pharmacological actions of fentanyl, we determined the effects of pretreating rats with the stable cell permeable superoxide dismutase-mimetic and free radical scavenger, Tempol (4-hydroxy-2,2,6,6-tetramethylpiperidine-N-oxyl), on the cardiorespiratory and analgesic effects elicited by injections of fentanyl. To support these studies we examined whether the cell permeable antioxidant, N-acetyl-L-cysteine methyl ester (L-NACme) modulated the ventilatory depressant effects of fentanyl (see Fig. 1 for depiction of Tempol, L-NAC and L-NACme structures)

### Materials and Methods

All animal studies were carried out in accordance with the National Institutes of Health Guide for the Care and Use of Laboratory Animals (NIH Publication No. 80.23) revised in 1996. The protocols were approved by the Institutional Animal Care and Use Committee at Galleon Pharmaceuticals, Inc., and Case Western Reserve University. Adult male Sprague-Dawley rats (body weights of 300 - 350 g) obtained from Harlan Laboratories, Inc. (Indianapolis, IN) were used for this study.

### Cardiorespiratory Measurements in Anesthetized Rats

The effects of bolus intravenous (IV) injections of vehicle (saline), Tempol (25, 50 or 100 mg/kg; stock solutions of Tempol of 100mg/ml in 0.9% saline were prepared freshly) and fentanyl (5 µg/kg) on heart rate (HR) and mean (MAP), diastolic (DBP) and systolic blood pressures (mean, systolic, diastolic), and frequency of breathing (Freq), tidal volume (TV) and minute ventilation (MV) were evaluated in anesthetized spontaneously breathing rats by methods described previously. In brief, rats were anesthetized with 2-2.5% isoflurane in compressed air. A length of PE-50 tubing (Instech Laboratories, Inc.) was inserted into a femoral vein to allow administration of drugs. The venous line was connected to saline filled three-way connector for fluid support (4ml/kg/h) and for IV administration of Tempol and fentanyl.

The cervical trachea was exposed ventrally, and a length of PE240 tubing (Instech Laboratories, Inc., Plymouth Meeting, PA, USA) was inserted and sutured in place. The tracheal tube was connected to a pneumotachometer (MLT1L, AD instruments, Inc., Colorado, USA) and a differential pressure transducer (FE141, AD instruments, Inc.) to measure respiratory flow via a T-shaped connector. The free end of the T-shaped connector was attached to the source of the isoflurane in compressed medical grade air. After the surgical procedures, animals were permitted to breath spontaneously on 1.5% isoflurane in throughout the experiment. The body temperature maintained at 37°C using a thermal blanket (Harvard Apparatus, Holliston, MA, USA). Respiratory flow was used to measure the Freq from the cyclic periods and integrated to measure TV. MV was calculated by multiplying Freq by TV.

A length of PE-50 tubing (Instech Laboratories, Inc.) was inserted into a femoral artery in order to continuously record cardiovascular parameters. The arterial catheter was connected to heparinized saline filled pressure transducer (SP844-28; Memscap Inc., North Carolina, USA) and arterial pressure signals were amplified using the bridge amplifier (FE221, AD Instruments, Inc). The arterial blood pressure waveform was used for measuring HR, DBP, SBP and MAP using the cyclic algorithms in the LabChart software. The respiratory flow and the blood pressure waveforms were digitized (PowerLab, AD Instruments, Inc.) and continuously recorded using LabChart 7 Pro Software (AD Instruments, Inc.). Note that after completion of the surgical procedures, baseline cardiorespiratory parameters were determined and recorded for 10 min once stable values occurred. The average of this 10 min period was determined and all subsequent (*i.e.,* pre- and post-injection) values were expressed as a percentage of this average.

### Ventilatory Recordings in Freely-Moving rats

Ventilatory parameters were continuously recorded in unrestrained freely-moving rats via a whole-body 12-chamber plethysmography system (PLY 3223; BUXCO Inc., Wilmington, NC, USA) as described previously. In brief, each rat was placed in an individual plexi-glass chamber and the venous line was attached to a swivel assembly on the roof of the chamber to allow drug injections. The respiratory flow waveform was derived from a pneumotachometer in the chamber wall. Respiratory flow, chamber temperature, and humidity were measured continuously and used to calculate TV using the Epstein and Epstein algorithm. The respiratory flow waveform cycle period was used to calculate Freq.

### Analgesia assessment by Tail-flick withdrawal Assay

The acute antinociceptive effects of Tempol and fentanyl were assessed on tail-withdrawal latency using a Tail-Flick Analgesia Meter (IITC Life Science Inc., USA) as described previously. This involved minor manual restraint while positioning the tail to apply a thermal stimulus (instrument setting of 50 to 75% active intensity), sufficient to induce a latency of tail withdrawal of 20 seconds (baseline values) prior to injection of drugs.

### Protocols

### Fentanyl-induced cardiorespiratory depression in the anesthetized model

The effects of Tempol on fentanyl-induced cardiorespiratory parameters were determined in anesthetized spontaneously breathing rats. After completion of the surgical procedures, baseline cardiorespiratory parameters were determined and the rats then received slow bolus injections of vehicle or Tempol (25, 50 or 100 mg/kg, IV). After 20 min, all of the rats received a bolus injection of fentanyl (5 µg/kg) and cardiorespiratory variables recorded for a further 15 min.

### Analgesia testing in conscious rats

To determine the effects of Tempol on analgesia status, baseline TF latencies were established and the rats then received bolus injections of vehicle (n=6) or 1, 10 or 100 mg/kg doses of Tempol (n= 6 rats per dose). TF latency was again tested 20, 40, 60, 90 and 120 min after injection of saline or Tempol. To determine the effects of Tempol on fentanyl-induced analgesia, baseline TF latencies were established at 10 AM and rats received an injection of fentanyl (25 µg/kg, IV). Five min later, the rats received injections of vehicle (1 ml/kg, n=6), or Tempol (100 mg/kg, n=6). TF latencies were tested 20, 40, 60, 90 and 120 min following injections of vehicle or Tempol. An injection of fentanyl (25 µg/kg, IV) was given again to both groups at 4 PM TF latencies were tested at 20, 40, 60, 90 and 120 min. To determine the effects of N-acetyl-L-cysteine methyl ester (L-NACme), on fentanyl-induced analgesia, baseline TF latencies were established at 10 AM and rats received an injection of fentanyl (25 µg/kg, IV). Five min later, the rats received injections of vehicle (1 ml/kg, n=6), or L-NACme (500 µmol/kg, IV, n=6). TF latencies were tested 20, 40, 60, 90, and 120 min following the injections of vehicle or Tempol. An injection of fentanyl (25 µg/kg, IV) was given again to both groups of rats at 4 PM and TF latencies were tested after 20, 40, 60, 90, and 120 min.

### Ventilatory Studies in Freely-Moving Rats

The effects of pretreatment with L-NACme on fentanyl-induced respiratory depression were evaluated in freely-moving rats. The rats were placed in the plethysmography chambers and after a period of acclimatization, they received an injection of vehicle (saline, n = 9) or L-NACme (500 µmol/kg, IV; n = 9) and after 10 min all rats received a bolus injection of fentanyl (25 µg/kg, IV). Ventilatory parameters were recorded for a further 15 min.

### Data Analyses

All data are presented as mean ± SEM and were evaluated using one way and two-way ANOVA plus Bonferroni corrections for multiple comparisons between means. Differences between means were considered to be significant at *P* < 0.05. Statistical analyses were performed using GraphPad Prism software (GraphPad Software, Inc., La Jolla, CA).

### Results

Fig. 2 summarizes the data showing that bolus injections of Tempol (25, 50 or 100 mg/kg, IV) elicited minimal changes in the frequency of breathing (Freq) and somewhat dose-dependent (relatively minor but sustained) increases in tidal volume (TV) and minute ventilation (MV). The key observation was that the subsequent injection of fentanyl (5 µg/kg, IV) in these rats elicited profound decreases in Freq, TV and MV in vehicle-treated rats but progressively and substantially smaller decreases in ventilatory parameters as the dose of Tempol was increased. Indeed, the fentanyl-induced change in MV in the rats treated with the 100 mg/kg dose of Tempol returned to the elevated levels seen prior to injection of fentanyl. As seen in Fig. 3, an injection of fentanyl (5 µg/kg, IV) markedly depressed the ventilatory waveform in a saline-treated rat but elicited progressively smaller responses in rats pre-injected with 25, 50 or 100 mg/kg of Tempol (each trace from separate rats).

Fig. 4 summarizes the data showing that the injections of Tempol (25, 50 or 100 mg/kg, IV) elicited pronounced dose-dependent decreases in mean (MAP), diastolic (DBP) and systolic (SBP) arterial blood pressures that were still sustained for the 100 mg/kg dose at the time fentanyl was injected (16 min post-Tempol). Tempol elicited somewhat minor but sustained decreases in heart rate (HR). As can be seen, the decreases in MAP, DBP and SBP were markedly diminished by Tempol, with the responses particularly reduced in size after administration of the 50 and 100 mg/kg doses of Tempol. As seen in Fig. 5, an injection of fentanyl (5 µg/kg, IV) markedly depressed arterial blood pressure (systolic and diastolic) in a saline-treated rat but elicited progressively smaller responses in rats pre-injected with 25, 50 or 100 mg/kg of Tempol (each trace from separate rats).

Fig. 6 summarizes the peak changes in cardiorespiratory parameters elicited by fentanyl (5 µg/kg, IV) in vehicle or Tempol-treated rats. It is readily seen that Tempol markedly diminishes the peak decreases in all of these parameters. A summary of the total ventilatory responses (all time points summed together) elicited by Vehicle or Tempol and the subsequent injections of fentanyl are summarized in Fig. 7. The pre-values (5 min) did not change compared to the prior baseline recordings (top panel). Tempol (25, 50 or 100 mg/kg, IV; designated T25, T50, T100) elicited significant increases in TV and MV but not Freq (middle panel). The fentanyl-induced decreases in Freq, TV and MV were substantially blunted by Tempol (bottom panel). A summary of the total cardiovascular responses (all time points summed together) elicited by Vehicle or Tempol and the subsequent injections of fentanyl are summarized in Fig. 8. The pre-values (5 min) did not change compared to the prior baseline recordings (top panel). Tempol (25, 50 or 100 mg/kg, IV; designated T25, T50, T100) elicited significant decreases in MAP, DBP, SBP and HR (middle panel). The fentanyl-induced decreases in MAP, DBP and SBP (but not HR) were substantially blunted by Tempol (bottom panel).

With respect to analgesia, we found that Tempol at various doses (1 mg/kg, n = 4; 10 mg/kg, n =4, or 100 mg/kg, n = 6) had no effects on tail-flick latencies of conscious rats for up to 2h post-administration (data not shown). As seen in Fig. 9, the analgesic effects (increases in tail-flick latency) in conscious rats elicited by a bolus injection of fentanyl (25 µg/kg, IV) were not affected by pretreatment with Tempol (100 mg/kg, IV) at a study commenced at 10 AM. As shown in Fig. 10, fentanyl given to the same rats at 4 PM (no drug pretreatments at this time) elicited significant analgesia of much reduced duration. The injection of Tempol in the 10 AM study did not affect this loss of analgesic effect of fentanyl at 4 PM.

Fig. 11 demonstrates that pretreatment with a large IV dose of L-NACme (500 µmol/kg, 88.61 mg/kg) had minimal effects on the ventilatory depressant effects elicited by a subsequent injection of fentanyl (25 µg/kg, IV). Fig. 12 shows that the 10 AM injection of L-NACme substantially blunted the development of tolerance to the analgesic responses elicited by the second injection of fentanyl given at 4 PM.

It was found that, in isoflurane-anesthetized rats (1) the systemic injection of Tempol elicited positive effects on minute ventilation in naïve isoflurane-anesthetized rats, (2) pretreatment with Tempol dose-dependently and markedly attenuated the cardiorespiratory depressant effects elicited by a bolus injection of fentanyl, and (3) a bolus injection of Tempol immediately reversed the pronounced cardiorespiratory depression elicited by an infusion of fentanyl. Other findings were that in conscious rats, (a) Tempol did not affect fentanyl-induced analgesia or (b) modify the decline in analgesia elicited by a second dose of fentanyl in the same rats. The ability of Tempol to profoundly affect the cardiorespiratory depressant effects of fentanyl without modulating fentanyl-induced analgesia would therefore seem to be by mechanisms that are independent of direct effects on opioid receptors.

In summary, we found that Tempol efficiently prevents fentanyl-induced depression of breathing and blood pressure in male rats. Although we did not establish that the effects of Tempol were due to its known ability to scavenge superoxide anion, our data do suggest that Tempol, which is a clinically approved drug for the treatment of alopecia, and related Tempol structures (see Fig. 13) may be repurposed as an intravenous agent to protect and/or reverse the negative cardiorespiratory effects of fentanyl while preserving analgesia in male and female children and adults.

## Claims

1. A composition comprising a nitroxide compound for use in the treatment of opioid induced respiratory depression in a subject in need thereof, by administering to said subject a therapeutically effective amount of said composition comprising a nitroxide compound, wherein the nitroxide compound comprises at least one of 4-hydroxy-2,2,6,6-tetramethylpiperidine-1-oxyl (Tempol), 2,2,6,6- tetramethylpiperidine-1-oxyl (Tempo), 4-amino-2,2,6,6-tetramethyl-1-piperidinyloxy (Tempamine); 4-oxo-2,2,6,6-tetramethyl-1-piperidinyloxy (4-oxo-Tempo), 4-carboxy- 2,2,6,6-tetramethyl-1-piperidinyloxy (4-carboxy-Tempo), 4-acetamido-2,2,6,6-tetramethyl-1- piperidinyloxy, 3-aminomethyl- 2,2,5,5-tetramethyl-1 -pyrrolidinyl-N-oxyl (3-aminomethyl-Proxyl), 3-cyano-2,2,5,5- tetramethyl-1-pyrrolidinyl-N-oxyl (3-cyano-Proxyl), 3-Carbamoyl-2,2,5,5-tetramethyl-1- pyrrolidinyl-N-oxyl (3-Carbamoyl-Proxyl), 3-Carboxy-2,2,5,5-tetramethyl-1-pyrrolidinyl-N- oxyl (3-Carboxy-Proxyl),or pharmaceutically acceptable salts, tautomers, or solvates thereof.

2. The composition for use according to claim 1, the treatment of opioid induced respiratory depression in a subject in need thereof being attenuation of opioid induced respiratory depression in a subject in need thereof, and the therapeutically effective amount of said composition comprising a nitroxide compound being an amount effective to attenuate the opioid induced respiratory depression.

3. The composition for use of claim 1 or 2, wherein the nitroxide compound comprises 4-hydroxy-2,2,6,6-tetramethylpiperidine-1-oxyl (Tempol) or pharmaceutically acceptable salts, tautomers, or solvates thereof.

4. The composition for use of claim 1 or 2, wherein the amount is an amount effective to increase tidal volume, increase respiratory frequency, increase minute ventilation, increase mean arterial blood pressure, increase diastolic blood pressure, and/or increase systolic blood pressure.

5. The composition for use of claim 1 or 2, wherein the opioid comprises at least one of alfentanil, buprenorphine, butorphanol, carfentanil, codeine, diamorphine, dextromoramide, dezocine, dihydrocodeine, fentanyl, hydrocodone, hydromorphone, levorphanol, meperidine, meptazinol, methadone, morphine, nalbuphine, nalorphine, opium, oxycodone, oxymorphone, pentazocine, propoxyphene, remifentanil, sufentanil, tapentadol, and tramadol, and pharmaceutically acceptable salts thereof.

6. The composition for use of claim 5, wherein the opioid is carfentanil, fentanyl, remifentanil, or sufentanil.

7. The composition for use of claim 1 or 2, wherein the composition is administered to the subject systemically.

8. A composition comprising:
an opioid capable of inducing respiratory depression in a subject and an amount of nitroxide compound effective to attenuate the opioid induced respiratory depression when the composition is administered to the subject, wherein the nitroxide compound comprises at least one of 4-hydroxy-2,2,6,6-tetramethylpiperidine-1-oxyl (Tempol), 2,2,6,6- tetramethylpiperidine-1-oxyl (Tempo), 4-amino-2,2,6,6-tetramethyl-1-piperidinyloxy (Tempamine); 4-oxo-2,2,6,6-tetramethyl-1-piperidinyloxy (4-oxo-Tempo), 4-carboxy- 2,2,6,6-tetramethyl-1-piperidinyloxy (4-carboxy-Tempo), 4-acetamido-2,2,6,6-tetramethyl-1- piperidinyloxy, 3-aminomethyl- 2,2,5,5-tetramethyl-1 -pyrrolidinyl-N-oxyl (3-aminomethyl-Proxyl), 3-cyano-2,2,5,5- tetramethyl-1-pyrrolidinyl-N-oxyl (3-cyano-Proxyl), 3-Carbamoyl-2,2,5,5-tetramethyl-1- pyrrolidinyl-N-oxyl (3-Carbamoyl-Proxyl), 3-Carboxy-2,2,5,5-tetramethyl-1-pyrrolidinyl-N- oxyl (3-Carboxy-Proxyl),or pharmaceutically acceptable salts, tautomers, or solvates thereof.

9. The composition of claim 8, wherein the opioid comprises at least one of alfentanil, buprenorphine, butorphanol, carfentanil, codeine, diamorphine, dextromoramide, dezocine, dihydrocodeine, fentanyl, hydrocodone, hydromorphone, levorphanol, meperidine, meptazinol, methadone, morphine, nalbuphine, nalorphine, opium, oxycodone, oxymorphone, pentazocine, propoxyphene, remifentanil, sufentanil, tapentadol, and tramadol, and pharmaceutically acceptable salts thereof.

10. The composition of claim 8, wherein the opioid is carfentanil, fentanyl, remifentanil, or sufentanil.

11. The composition of claim 8, wherein the nitroxide compound comprises 4-hydroxy-2,2,6,6-tetramethylpiperidine-1-oxyl (Tempol) or pharmaceutically acceptable salts, tautomers, or solvates thereof.

## Patentansprüche

1. Eine Zusammensetzung, die eine Nitroxidverbindung zur Verwendung bei der Behandlung einer durch Opioide verursachten Atemdepression bei einem Patienten umfasst, indem dem Patienten eine therapeutisch wirksame Menge der Zusammensetzung verabreicht wird, die eine Nitroxidverbindung umfasst, wobei die Nitroxidverbindung mindestens eines von 4-Hydroxy-2,2,6,6-tetramethylpiperidin-1-oxyl (Tempol), 2,2,6,6-Tetramethylpiperidin-1-oxyl (Tempo), 4-Amino-2,2,6,6-tetramethyl-1-piperidinyloxy (Tempamine); 4-Oxo-2,2,6,6-tetramethyl-1-piperidinyloxy (4-Oxo-Tempo), 4-Carboxy-2,2,6,6-tetramethyl-1-piperidinyloxy (4-Carboxy-Tempo), 4-Acetamido-2,2,6,6-tetramethyl-1-piperidinyloxy, 3-Aminomethyl-2,2,5,5-tetramethyl-1-pyrrolidinyl-N-oxyl (3-Aminomethyl-Proxyl), 3-Cyano-2,2,5,5-tetramethyl-1-pyrrolidinyl-N-oxyl (3-Cyano-Proxyl), 3-Carbamoyl-2,2,5,5-tetramethyl-1-pyrrolidinyl-N-oxyl (3-Carbamoyl-Proxyl), 3-Carboxy-2,2,5,5-tetramethyl-1-pyrrolidinyl-N-oxyl (3-Carboxy-Proxyl), oder pharmazeutisch verträgliche Salze, Tautomere oder Solvate davon umfasst.

2. Die Zusammensetzung zur Verwendung gemäß Anspruch 1, wobei die Behandlung der durch Opioide verursachten Atemdepression bei einem Patienten die Abschwächung der durch Opioide verursachten Atemdepression bei einem Patienten ist und die therapeutisch wirksame Menge der Zusammensetzung, die eine Nitroxidverbindung umfasst, eine Menge ist, die wirksam ist, um die durch Opioide verursachte Atemdepression abzuschwächen.

3. Die Zusammensetzung zur Verwendung gemäß Anspruch 1 oder 2, wobei die Nitroxidverbindung 4-Hydroxy-2,2,6,6-tetramethylpiperidin-1-oxyl (Tempol) oder pharmazeutisch verträgliche Salze, Tautomere oder Solvate davon umfasst.

4. Die Zusammensetzung zur Verwendung nach Anspruch 1 oder 2, wobei die Menge eine Menge ist, die wirksam ist, um das Atemzugvolumen zu erhöhen, die Atemfrequenz zu erhöhen, die Minutenventilation zu erhöhen, den mittleren arteriellen Blutdruck zu erhöhen, den diastolischen Blutdruck zu erhöhen und/oder den systolischen Blutdruck zu erhöhen.

5. Die Zusammensetzung zur Verwendung gemäß Anspruch 1 oder 2, wobei das Opioid mindestens eines von Alfentanil, Buprenorphin, Butorphanol, Carfentanil, Codein, Diamorphin, Dextromoramid, Dezocin, Dihydrocodein, Fentanyl, Hydrocodon, Hydromorphon, Levorphanol, Meperidin, Meptazinol, Methadon, Morphin, Nalbuphin, Nalorphin, Opium, Oxycodon, Oxymorphon, Pentazocin, Propoxyphen, Remifentanil, Sufentanil, Tapentadol und Tramadol, sowie deren pharmazeutisch verträgliche Salze umfasst.

6. Die Zusammensetzung zur Verwendung gemäß Anspruch 5, wobei das Opioid Carfentanil, Fentanyl, Remifentanil oder Sufentanil ist.

7. Die Zusammensetzung zur Verwendung nach Anspruch 1 oder 2, wobei die Zusammensetzung dem Patienten systemisch verabreicht wird.

8. Eine Zusammensetzung, umfassend:
ein Opioid, das bei einem Patienten eine Atemdepression verursachen kann, und eine Menge einer Nitroxidverbindung, die wirksam ist, um die durch das Opioid verursachte Atemdepression zu mildern, wenn die Zusammensetzung dem Patienten verabreicht wird, wobei die Nitroxidverbindung mindestens eines von 4-Hydroxy-2,2,6,6-tetramethylpiperidin-1-oxyl (Tempol), 2,2,6,6-Tetramethylpiperidin-1-oxyl (Tempo), 4-Amino-2,2,6,6-tetramethyl-1-piperidinyloxy (Tempamin); 4-Oxo-2,2,6,6-tetramethyl-1-piperidinyloxy (4-Oxo-Tempo), 4-Carboxy-2,2,6,6-tetramethyl-1-piperidinyloxy (4-Carboxy-Tempo), 4-Acetamido-2,2,6,6-tetramethyl-1-piperidinyloxy, 3-Aminomethyl-2,2,5,5-tetramethyl-1-pyrrolidinyl-N-oxyl (3-Aminomethyl-Proxyl), 3-Cyano-2,2,5,5-tetramethyl-1-pyrrolidinyl-N-oxyl (3-Cyano-Proxyl), 3-Carbamoyl-2,2,5,5-tetramethyl-1-pyrrolidinyl-N-oxyl (3-Carbamoyl-Proxyl), 3-Carboxy-2,2,5,5-tetramethyl-1-pyrrolidinyl-N-oxyl (3-Carboxy-Proxyl), oder pharmazeutisch verträgliche Salze, Tautomere oder Solvate davon umfasst.

9. Die Zusammensetzung nach Anspruch 8, wobei das Opioid mindestens eines von Alfentanil, Buprenorphin, Butorphanol, Carfentanil, Codein, Diamorphin, Dextromoramid, Dezocin, Dihydrocodein, Fentanyl, Hydrocodon, Hydromorphon, Levorphanol, Meperidin, Meptazinol, Methadon, Morphin, Nalbuphin, Nalorphin, Opium, Oxycodon, Oxymorphon, Pentazocin, Propoxyphen, Remifentanil, Sufentanil, Tapentadol und Tramadol und pharmazeutisch verträgliche Salze davon umfasst.

10. Die Zusammensetzung nach Anspruch 8, wobei das Opioid Carfentanil, Fentanyl, Remifentanil oder Sufentanil ist.

11. Die Zusammensetzung nach Anspruch 8, wobei die Nitroxidverbindung 4-Hydroxy-2,2,6,6-tetramethylpiperidin-1-oxyl (Tempol) oder pharmazeutisch verträgliche Salze, Tautomere oder Solvate davon umfasst.

## Revendications

1. Composition comprenant un composé nitroxyde pour utilisation dans le traitement de dépression respiratoire induite par opioïde chez un sujet ayant besoin de celui-ci, en administrant audit sujet une quantité thérapeutiquement efficace de ladite composition comprenant un composé nitroxyde, dans laquelle le composé nitroxyde comprend au moins l'un parmi 4-hydroxy-2,2,6,6-tétraméthylpipéridine-1-oxyle (Tempol), 2,2,6,6-tétraméthylpipéridine-1-oxyle (Tempo), 4-amino-2,2,6,6-tétraméthyl-1-pipéridinyloxy (Tempamine) ; 4-oxo-2,2,6,6-tétraméthyl-1-pipéridinyloxy (4-oxo-Tempo), 4-carboxy-2,2,6,6-tétraméthyl-1-pipéridinyloxy (4-carboxy-Tempo), 4-acétamido-2,2,6,6-tétraméthyl-1-pipéridinyloxy, 3-aminométhyl-2,2,5,5-tétraméthyl-1-pyrrolidinyl-N-oxyle (3-aminométhyl-Proxyle), 3-cyano-2,2,5,5-tétraméthyl-1-pyrrolidinyl-N-oxyle (3-cyano-Proxyle), 3-Carbamoyl-2,2,5,5-tétraméthyl-1-pyrrolidinyl-N-oxyle (3-Carbamoyl-Proxyle), 3-Carboxy-2,2,5,5-tétraméthyl-1-pyrrolidinyl-N-oxyle (3-Carboxy-Proxyle), ou des sels, tautomères ou solvates pharmaceutiquement acceptables de ceux-ci.

2. Composition pour utilisation selon la revendication 1, le traitement de dépression respiratoire induite par opioïde chez un sujet ayant besoin de celui-ci étant une atténuation de dépression respiratoire induite par opioïde chez un sujet ayant besoin de celui-ci, et la quantité thérapeutiquement efficace de ladite composition comprenant un composé nitroxyde étant une quantité efficace pour atténuer la dépression respiratoire induite par opioïde.

3. Composition pour utilisation selon la revendication 1 ou 2, dans laquelle le composé nitroxyde comprend 4-hydroxy-2,2,6,6-tétraméthylpipéridine-1-oxyle (Tempol) ou des sels, tautomères ou solvates pharmaceutiquement acceptables de celui-ci.

4. Composition pour utilisation selon la revendication 1 ou 2, dans laquelle la quantité est une quantité efficace pour augmenter un volume courant, augmenter une fréquence respiratoire, augmenter une ventilation minute, augmenter une pression artérielle moyenne, augmenter une pression artérielle diastolique et/ou augmenter une pression artérielle systolique.

5. Composition pour utilisation selon la revendication 1 ou 2, dans laquelle l'opioïde comprend au moins l'un parmi alfentanil, buprénorphine, butorphanol, carfentanil, codéine, diamorphine, dextromoramide, dézocine, dihydrocodéine, fentanyl, hydrocodone, hydromorphone, lévorphanol, mépéridine, meptazinol, méthadone, morphine, nalbuphine, nalorphine, opium, oxycodone, oxymorphone, pentazocine, propoxyphène, rémifentanil, sufentanil, tapentadol et tramadol, et des sels pharmaceutiquement acceptables de ceux-ci.

6. Composition pour utilisation selon la revendication 5, dans laquelle l'opioïde est carfentanil, fentanyl, rémifentanil ou sufentanil.

7. Composition pour utilisation selon la revendication 1 ou 2, dans laquelle la composition est administrée au sujet de manière systémique.

8. Composition comprenant :
un opioïde capable d'induire une dépression respiratoire chez un sujet et une quantité de composé nitroxyde efficace pour atténuer la dépression respiratoire induite par opioïde lorsque la composition est administrée au sujet, dans laquelle le composé nitroxyde comprend au moins l'un parmi 4-hydroxy-2,2,6,6-tétraméthylpipéridine-1-oxyle (Tempol), 2,2,6,6-tétraméthylpipéridine-1-oxyle (Tempo), 4-amino-2,2,6,6-tétraméthyl-1-pipéridinyloxy (Tempamine) ; 4-oxo-2,2,6,6-tétraméthyl-1-pipéridinyloxy (4-oxo-Tempo), 4-carboxy-2,2,6,6-tétraméthyl-1-pipéridinyloxy (4-carboxy-Tempo), 4-acétamido-2,2,6,6-tétraméthyl-1-pipéridinyloxy, 3-aminométhyl-2,2,5,5-tétraméthyl-1-pyrrolidinyl-N-oxyle (3-aminométhyl-Proxyle), 3-cyano-2,2,5,5-tétraméthyl-1-pyrrolidinyl-N-oxyle (3-cyano-Proxyle), 3-Carbamoyl-2,2,5,5-tétraméthyl-1-pyrrolidinyl-N-oxyle (3-Carbamoyl-Proxyle), 3-Carboxy-2,2,5,5-tétraméthyl-1-pyrrolidinyl-N-oxyle (3-Carboxy-Proxyle), ou des sels, tautomères ou solvates pharmaceutiquement acceptables de ceux-ci.

9. Composition selon la revendication 8, dans laquelle l'opioïde comprend au moins l'un parmi alfentanil, buprénorphine, butorphanol, carfentanil, codéine, diamorphine, dextromoramide, dézocine, dihydrocodéine, fentanyl, hydrocodone, hydromorphone, lévorphanol, mépéridine, meptazinol, méthadone, morphine, nalbuphine, nalorphine, opium, oxycodone, oxymorphone, pentazocine, propoxyphène, rémifentanil, sufentanil, tapentadol et tramadol, et des sels pharmaceutiquement acceptables de ceux-ci.

10. Composition selon la revendication 8, dans laquelle l'opioïde est carfentanil, fentanyl, rémifentanil ou sufentanil.

11. Composition selon la revendication 8, dans laquelle le composé nitroxyde comprend 4-hydroxy-2,2,6,6-tétraméthylpipéridine-1-oxyle (Tempol) ou des sels, tautomères ou solvates pharmaceutiquement acceptables de celui-ci.
